# EUROPEAN PATENT APPLICATION

(11) **EP 3 301 085 A1**
(43) Date of publication of application: **04.04.2018**
(21) Application number: 16191348.8
(22) Date of filing: 29.09.2016
(51) Int. Cl.: C07C 59/72, C07C 59/74, C07C 59/90, C07C 229/34, C07C 235/42, C07C 251/40, C07C 251/48, C07C 251/60, C07C 259/06, C07C 279/14, C07C 279/24, C07C 271/20, C07C 271/22, C07C 323/41, C07C 403/20, C07D 215/12, C07D 241/04, C07D 215/28, A61K 31/201, A61P 35/00

(54) **RETINOID DERIVATIVES WITH ANTITUMOR ACTIVITY**

(71) Applicant: BIOGEM S.CA.R.L., 83031 Ariano Irpino (AV) (IT)
(72) Inventor: PISANO, Claudio, 04011 Aprilla (LT) (IT); DALLAVALLE, Sabrina, 20871 Vimercate (MB) (IT); CINCINELLI, Raffaella, 24041 Bremate (BG) (IT); MERLINI, Lucio, 55049 Viareggio (LU) (IT)
(74) Representative: Ghirardi, Valeria

(57) **Abstract**

The present invention relates to new compounds of formula (I) and to pharmaceutical compositions containing them:

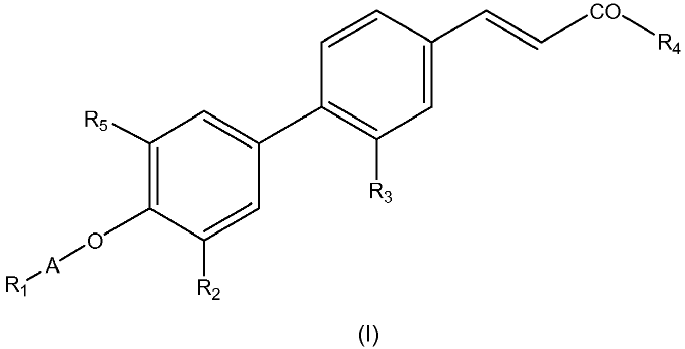

The use of such compounds for the treatment of cancer and other diseases related to altered angiogenesis, such as arthritic pathology, diabetic retinopathy, psoriasis and chronic inflammatory disease, is also within the scope of the present invention.

## Description

### FIELD OF THE INVENTION

The present invention relates to the field of pharmaceutical compounds, in particular retinoid derivatives.

### BACKGROUND OF THE INVENTION

Vitamin A and its biologically active derivatives, retinal and retinoic acid, play an important role in vision, are necessary in the reproductive system, act as morphogenic agents during embryonic growth and regulate the growth and differentiation of a vast range of cell types at the basis of the growth of an organism (M.Sporn, A.Roberts, D.Goodman, The Retinoids, Raven Press, New York 1994). The biological action of retinoic acid and its derivatives is mediated by the interaction with nuclear receptors belonging to two families: the first named RAR (retinoic acid receptor) and the second named RXR (retinoid X receptor) (M.A. Asson-Batres, C. Rochette-Egly, The Biochemistry of Retinoic Acid Receptors I: Structure, Activation, and Function at the Molecular Level, Springer, Dordrecht, 2014).

Each family is divided into 3 subtypes (α, β, γ) coded by three different genes. All-Trans-Retinoic Acid (ATRA) binds to RAR and RXR, whilst 9-cis RA binds only to RXR.

Retinoids, whether natural or synthetic vitamin A analogues, exert a great influence over cellular proliferation, differentiation and apoptosis: these properties are amply exploited in the control of tumoral and dermatological pathologies (B.C. Das et al. Bioorganic & Medicinal Chemistry 22 (2014) 673-683).

It is well known that the growth of a primary tumour is favoured by good vascularization of the tumour tissue. An adequate supply of oxygen and nutrients promotes rapid growth of the tumour itself.

It has been demonstrated that the extent of angiogenesis can be an extremely negative factor in the prognosis of neoplasms (J.A. van der Zee et al., Eur. J. Cancer, 47 (2011) 2576 - 2584).

It is also known, in the neoplastic field, that a fundamental stage in the biology of the tumour cell is the acquisition of metastasising capability.

The tumour cells that metastasise are able to lose adherence to the surrounding structures, invade blood and lymphatic vessels and colonise other tissues at a distance where they can continue to reproduce themselves.

Metastasising is also a critical event in the clinical history of the disease, being the main cause of death due to cancer. It is closely associated with and facilitated by the presence of vascular tissue in the tumour site or adjacent areas.

The migration of tumour cells across the surrounding structures enables the cells to reach the intratumoral blood vessels, whether pre-existing or formed by neo-angiogenesis, and thus reach the bloodstream (Ray JM., Stetler-Stevenson WG; Eur. Respir. J., 7(11):2062-72, 1994; Stetler-Stevenson WG, Liotta LA, Kleiner DE Jr; FASEB J., 7(15):1434-41, 1993; Sun Y, Ma, L. Trends in Pharmacological Sciences, 36, 349-359, 2015).

Retinoids useful for treating cancer are already known. Acute promielocytic leukemia (APL) can be effectively eradicated by retinoid signaling combined with chemotherapy, and therefore forms the prototype for retinoid-based therapies.

Various cancers other than APL are already being treated with retinoid-based therapies, and several are undergoing clinical evaluation. For several neoplastic diseases, more than one retinoid is being evaluated and many clinical studies are underway to assess their efficacy.

In clinical trials, ATRA and other retinoids alone have shown limited therapeutic success (Connolly RM, Nguyen NK, Sukumar S. Cancer Res 2013;19:1651-9) that may be attributed, in part, to frequent epigenetic silencing of the retinoic acid receptor (RAR)-β (Tang X.H. et al. Annu. Rev. Pathol 2011;6:345-645). It has been shown that histone deacetylase (HDAC) inhibitors cause re-expression of RAR-β and sensitize the cells to treatment (Sirchia SM, et al. Oncogene 2000;19:1556-63). Therefore combination of retinoids with HDAC inhibitors has been investigated (Pili R, et al. Br. J. Cancer 2012;106:77-84; Raffoux E. et al. Oncotarget 2010;1:34-4.)

Recently, much interest has been devoted to atypical retinoids or retinoid-related molecules (RRMs), a name that reflects the fact that they exert their anticancer activities independently of the transactivation of the retinoid receptors (RARs, subtypes α, β, and γ; and RXRs, subtypes α, β, and γ). RARs and RXRs are members of the nuclear hormone receptor superfamily of ligand-responsive transcription factors, which mediate the multifarious actions of natural and synthetic retinoids in embryo and throughout life.

However, some compounds of this class, containing the adamantyl group, called adamantyl retinoids (Wanka et al. Chem. Rev. 2013, 113, 3516-3604), are known to bind to RARs, such as (6-[(3-adamant-1-yl)-4-hydroxy-phenyl]-2-naphthoic acid, CD437, also called AHPN), which is a RARγ agonist. Other analogs of CD437, such as Adarotene (AHPC), which also binds RARγ and is an apoptogenic agent stronger than CD437, 5-Cl-AHPN and 3-Cl-AHPC, lack RAR transactivation activity while preserving the induction of growth arrest and apoptotic activity in a variety of cancer cell lines (Dawson et al. J. Med. Chem. 2004, 47, 3518). Moreover, 3-Cl-AHPC binds the nuclear hormone receptor small heterodimer partner (SHP) and modulates SHP interaction with the Sin3A repressor (Farhana et al., Mol. Cancer Ther. 2009, 8, 1625). While still unclear, the mechanism of RRM-mediated cell death, in particular the apoptosis induced by CD437, appears to be largely dependent on cell type. Evidences for caspase-dependent and independent mechanisms via the intrinsic and extrinsic pathways have been provided for these compounds (Lopez-Hernandez et al., Cell Death Differ. 2004, 11, 154- 164. Inhibition of IGF-1R and Wnt/β-Catenin pathways are also involved (L. Farhana et al. J. of Oncology, 2012, 796729).

WO9703682 discloses the use of the above mentioned AHPN compound for treating cancer.

WO9801132 and WO0156563 disclose adamantyl containing retinoid compounds.

WO2010072727, WO2007000383 and WO0311808 disclose retinoid derivatives with cytotoxic and/or antitumoral properties. WO2010106135 discloses retinoid derivatives for the treatment of ovarian carcinoma. WO2008077772 discloses a combination comprising an atypical retinoid and a platinum anticancer agent to inhibit tumor growth and tumor migration.

Despite the progress made in recent years, the pharmacological research concerning the discovery of new drugs for the treatment of tumor diseases is still one of the most active fields.

Indeed, there is still a strong need of new compounds capable of blocking or interfering with the tumour diseases.

### SUMMARY OF THE INVENTION

It has now been found that new compounds of formula (I), or the pharmaceutically acceptable salts thereof, can be successfully used in the treatment of cancer and other diseases related to altered angiogenesis.

It is an object of the present invention a compound having the following formula (I): 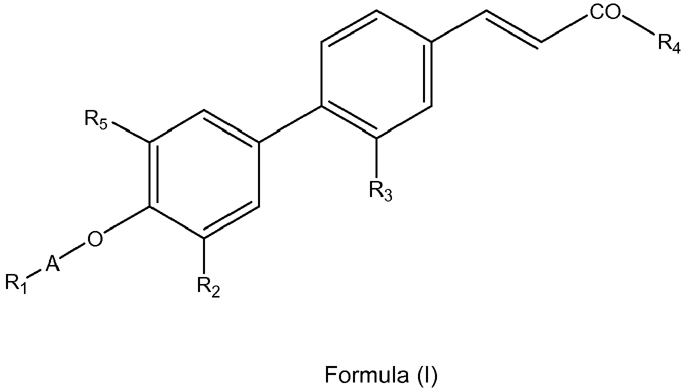 wherein:
A is selected from the group consisting of: alkylene, alkenylene and alkynylene, or is absent,
R₁ is selected from the group consisting of: H, OH, COOH, COO-alkyl, CONHOH, CONH₂, COCF₃, SH, SCOCH₃, CONHalkyl-S-S-alkyl, CONHalkyl-S-S-alkyl-NH₂, CONHalkyl-S-S-alkyl-NHCO-C(=NOH)alkylaryl wherein said aryl is optionally substituted with OH or halogen, COalkylCONHalkyl-S-S-alkyl-NHCO-C(=NOH)alkylaryl wherein said aryl is optionally substituted with OH or halogen, O-4-retinoic acid and O-4-N-(4-hydroxyphenyl)retinamide,
R₂ is selected from the group consisting of: H, alkyl, OH, O-alkyl, O-alkenyl, halogen, CN, CHO, COalkyl, COOH, COOalkyl and CONH₂,
R₃ is selected from the group consisting of: H, alkyl, alkenyl, oxo-alkenyl, hydroxyiminoalkenyl, OH, alkoxy, cycloalkyloxy, arylalcoxy, heterocycloalcoxy, carboxyalcoxy, CH₂R₆, COR₇, CN, CH=NOH, CH=NOalkylCOOH, CH=NOaryl and CH=NOalkylaryl,
   wherein R₆ is selected from the group consisting of: OH, alkoxy, NH₂, NH-alkyl, N-dialkyl, NHCOOalkyl, NHCOalkyl, aryl, heterocycloalkyl, guanidino optionally substituted with an alkyl or a COOalkyl group, biguanido optionally substituted with an alkyl or an arylalkyl group,
   and wherein R₇ is selected from the group consisting of: H, OH, O-alkyl, NH₂, NHOH, NHaryl, NHalkyl optionally terminally substituted with NH₂ or CONHOH,
R₄ is selected from the group consisting of: OH, O-alkyl, NH₂, NHaryl, NHalkyl, Ndialkyl, NHOH, NHNH₂ and CF₃, and
R₅ is selected from the group consisting of: alkyl, cycloalkyl, azacycloalkyl, oxacycloalkyl, aryl, heteroaryl, alkylamminomethyl, arylakylaminomethyl, heteroarylalkylaminomethyl, wherein each of said groups can be optionally substituted with OH, Oalkyl, halogen, NO₂ or NH₂, and each of cycloalkyl, azacycloalkyl, oxacycloalkyl, aryl, heteroaryl group can be substituted with alkyl,
   any nitrogen atom optionally bearing a protective group,
   wherein alkyl is a C₁-C₂₀ linear or branched alkyl group,
   alkenyl is a C₂-C₂₀ linear or branched alkyl group,
   cycloalkyl is a saturated or partially unsaturated C₃-C₁₀ carbocyclic group comprising one or more condensed rings,
   aryl is an aromatic group comprising one or more condensed aromatic rings,
   heterocycloalkyl and heteroaryl are said cycloalkyl or aryl rings containing one or more heteroatoms selected from the group consisting of N, O and S,
   amino is a group NR'R" wherein each of R' and R" can independently be H or alkyl,
   provided that when R₃ is H, R₁ is not H,
   the enantiomers, diastereoisomers, and mixtures thereof,
   or a pharmaceutically acceptable salt, hydrate or solvate thereof,
   provided that it is not the compound (E)-3-(3'-Adamantan-1-yl-4'-carbamoylmethoxybiphenyl-4-yl)-acrylic acid.

It has also been found that the combination of compounds of formula (I) with known chemotherapeutic agents can have a synergistic antitumor effect.

Furthermore, compounds of formula (I) are effective in tumoral cells resistant to other known chemotherapeutic drugs such as, for example, adarotene.

A process for the preparation of the compound of formula (I), as will be better defined below, is also an object of the present invention.

A further object of the invention is a pharmaceutical composition containing as active ingredient a compound of formula (I) and at least one pharmaceutically acceptable vehicle and/or excipient. Such composition can also contain one or more further chemotherapeutic drugs.

It is also an object of the invention the use of the compound of formula (I) as a medicament.

A further object of the present invention is the use of a compound of formula (I) for the treatment of a disease related to altered angiogenesis. In a preferred embodiment, said disease is selected from the group consisting of arthritic pathology, tumour, metastatization, diabetic retinopathy, psoriasis and chronic inflammatory disease.

### Detailed description of the invention

### Definitions

The term "alkyl" refers to linear or branched alkyl groups having from 1 to 20 carbon atoms, preferably having 1 to 12 carbon atoms.

The term "cycloalkyl" refers to a saturated or partially unsaturated carbocyclic group of 3 to 10 carbon atoms. An aromatic group is not intended. The cycloalkyl group can comprise a single ring or multiple condensed rings. Examples of "C₃-C₁₀₋cycloalkyl" include cyclopropyl, cyclobutyl, cyclopentyl, cyclohexyl, norbornyl, adamantyl and the like.

The term "azacycloalkyl" refers to compounds where one or more nitrogen atoms substitute for carbons in the "cycloalkyl" group.

The terms "heterocycloalkyl" and "heterocycle" refer to a saturated or partially unsaturated (but not aromatic) five-, six- or seven-membered ring containing one or more nitrogen, oxygen or sulfur atoms; the rings may be substituted with one o more groups selected from alkyl, hydroxyl and carboxyl. Preferred heterocycloalkyl include pyrrolidine, piperidine, piperazine, morpholine and tetrahydropyran.

The term "aryl" refers to aromatic rings, for example phenyl, naphthyl, anthracenyl, optionally substituted with OH, Oalkyl, halogen, NO₂ or NH₂.

The term "heteroaryl" refers to heteroaromatic rings, for example furan, thiophene, pyridine, pyrimidine, quinoline, quinazoline, optionally substituted with OH, Oalkyl, halogen, NO₂ or NH₂.

The term "amino" refers to a group NRR' wherein each of R and R' can independently be H or alkyl.

The term "aminocarbonylalkyl" refers to an alkyl group substituted by an aminocarbonyl moiety.

The term "aminocarbonyl" refers to a group of formula -NR'R"CO- wherein each of R' and R" can independently be H or alkyl.

### Figures

Figure 1. Efficacy of compound 2 (RC1120) in an orthotopic xenograft model of human hepatocellular carcinoma.
Figure 2. Efficacy of compound 22 (RC1375) in an human glioblastoma orthotopic model. Mice (n = 5) were administered intraperitoneally with the compound or vehicle only.
Figure 3. Efficacy of compound 8 (AB472) in an orthotopic model of human mesothelioma named MM487. Compound 8 was administered alone or in combination with cisplatin.

An alkyl group can be for example methyl, ethyl, n-propyl, i-propyl, n-butyl, t-butyl. It can be linear or branched.

An alkylene group is preferably methylene, ethylene, trimethylene, propylene, tetramethylene or dimethylethylene.

The halogen is selected from the group consisting of: fluorine (F), chlorine (CI), bromine (Br) and iodine (I).

Any nitrogen group can be protected. The protective group is preferably selected from tert-butoxycarbonyl, benzyloxycarbonyl and 9-fluorenylmethyloxycarbonyl.

In a preferred embodiment of the present invention, the compound of formula (I) has a cycloalkyl as R₅, more preferably it has an adamantyl group as R₅.

In another preferred embodiment, R₄ is OH or O-alkyl.

In another preferred embodiment, A is an alkyl and R₁ is H. In a further preferred embodiment, A is absent and R₁ is H.

The pharmaceutical acceptable salts of the compound of formula (I) are included in the scope of the invention.

Pharmaceutical acceptable salts are salts which retain the biological activity of the base and are derived from such known pharmacologically acceptable acids such as, e.g., hydrochloric, hydrobromic, sulfuric, phosphoric, nitric, fumaric, succinic, oxalic, malic, tartaric, maleic, citric, methanesulfonic or benzoic acid and others commonly used in the art. A preferred salt is a salt of the trifluoroacetic acid.

Also, pharmaceutically acceptable hydrates or solvates of the compound of formula (I) are included in the scope of the present invention. In particular, they can be any hydrate or solvate commonly used in the art.

Preferred compounds according to the present invention are:
3-[3'-Adamantan-1-yl-2-(tert-butoxycarbonylamino-methyl)-4'-hydroxy-biphenyl-4-yl]-acrylic acid (1) (AB461)
3-(3'-adamantan-1-yl-2-aminomethyl-4'-hydroxy-biphenyl-4-yl)-acrylic acid trifluoroacetate (2) (RC 1120)
3-(3'-adamantan-1-yl-4'-hydroxy-2-morpholin-4-ylmethyl-biphenyl-4-yl)-acrylic acid hydrochloride (3) (RC 1151)
3-(3'-adamantan-1-yl-4'-hydroxy-3-(4-methyl-piperazin-1-ylmethyl)-biphenyl-4-yl)-acrylic acid hydrochloride (4) (RC 1152)
3-[3'-adamantan-1-yl-2-(4-amino-butylcarbamoyl)-4'-hydroxy-biphenyl-4-yl]-acrylic acid trifluoroacetate (5) (RC 1193)
3-(3'-adamantan-1-yl-2-ditert-butoxycarbonylguanidinomethyl-4'-hydroxy-biphenyl-4-yl)-acrylic acid trifluoroacetate (6) (RC 1190)
3-(3'-adamantan-1-yl-2-guanidinomethyl-4'-hydroxy-biphenyl-4-yl)-acrylic acid trifluoroacetate (7) (RC 1191)
3-[3'-adamantan-1-yl-4'-hydroxy-2-(hydroxyimino-methyl)-biphenyl-4-yl]-acrylic acid (8) (AB 472)
3-[3'-adamantan-1-yl-4'-hydroxy-2-(tert-butoxyimino-methyl)-biphenyl-4-yl]-acrylic acid (9) (AB 473)
3-[3'-adamant-1-yl-4'-hydroxy-2-methoxyimino-methyl-biphenyl-4-yl]-acrylic acid. (10) (AB 474)
3-[3'-Adamantan-1-yl-2-(4-tert-butoxycarbonylamino-butylcarbamoyl)-4'-hydroxy-biphenyl-4-yl]-acrylic acid (11) (RC1189)
3-[3'-adamantan-1-yl-2-(tert-butoxycarbonylaminomethyl)-4'-methoxy-biphenyl-4-yl]-acrylic acid (12) (RC 1171)
3'-Adamantan-1-yl-4-(2-carboxy-vinyl)-4'-methoxy-biphenyl-2-yl-methyl-ammonium trifluoroacetate (13) (RC 1174)
3-[3'-Adamant-1-yl-4'-hydroxy-2-carboxymethoxyimino-methyl-biphenyl-4-yl]-acrylic acid. (14) (AB 486)
3-[3'-Adamantan-1-yl-4'-methoxy-2-(formyl)-biphenyl-4-yl]-acrylic acid (15) (RC 1237)
3-[3'-Adamantan-1-yl-4'-methoxy-2-(hydroxyimino-methyl)-biphenyl-4-yl]-acrylic acid (16) (RC 1230)
3-[3'-Adamantan-1-yl-4'-methoxy-2-(hydroxymethyl)-biphenyl-4-yl]-acrylic acid (17) (RC 1243)
3-[3'-Adamantan-1-yl-4'-methoxy-2-(3-oxo-but-1-enyl)-biphenyl-4-yl]-acrylic acid (18) (RC1236)
3-[3'-Adamantan-1-yl-2-(3-hydroxyimino-but-1-enyl)-4'-methoxy-biphenyl-4-yl]-acrylic acid (19) (RC1238)
3-(3'-Adamantan-1-yl-2-carbamoyl-4'-methoxy-biphenyl-4-yl)-acrylic acid (20) (RC 1277)
3-[3'-Adamantan-1-yl-4'-(3-hydroxycarbamoyl-propoxy)-biphenyl-4-yl]-acrylic acid (21) (RC 1315)
3-[3'-Adamantan-1-yl-4'-(4-hydroxycarbamoyl-butoxy)-biphenyl-4-yl]-acrylic acid (22) (RC 1375)
3-[3'-Adamantan-1-yl-4'-(7-hydroxycarbamoyl-heptyloxy)-biphenyl-4-yl]-acrylic acid (23) (RC 1268)
2-(2-{4-[3-Adamantan-1-yl-4'-(2-carboxy-vinyl)-biphenyl-4-yloxy]-butyrylamino}-ethyldisulfanyl)-ethyl-ammonium; trifluoroacetate (24) (RC 1363)
3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propoxy]-biphenyl-4-yl}-acrylic acid (25) (RC 1338)
3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propionyloxy]-biphenyl-4-yl}-acrylic acid (26) (AB 514)
3-[3'-Adamantan-1-yl-4'-hydroxy-2-(7-hydroxycarbamoyl-heptylcarbamoyl)-biphenyl-4-yl]-acrylic acid (27) (RC 1207)
3-[3'-Adamantan-1-yl-4'-(4-carboxy-butoxy)-biphenyl-4-yl]-acrylic acid (28)
3-(3'-Adamantan-1-yl-4'-hydroxycarbamoylmethoxybiphenyl-4-yl)-acrylic acid (MIR002) (29)
3-[3'-(1,5-Diaza-bicyclo[3.3.1]non-9-yl)-4'-(4-hydroxycarbamoyl-butoxy)-biphenyl-4-yl]-acrylic acid (30)
3-[4'-(4-Hydroxycarbamoyl-butoxy)-3'-(1,3,5,7-tetraaza-tricyclo[3.3.1.13,7]dec-2-yl)-biphenyl-4-yl]-acrylic acid (31)
3-[4'-(4-Hydroxycarbamoyl-butoxy)-3'-(1-methyl-cyclohexyl)-biphenyl-4-yl]-acrylic acid (32)
3-Phenyl-4'-(4-hydroxycarbamoyl-butoxy)-biphenyl-4-yl]-acrylic acid (33)
3-(4'-(4-Hydroxycarbamoyl-butoxy)-3'-{[(naphthalen-2-ylmethyl)-amino]-methyl}-biphenyl-4-yl)-acrylic acid (34)
3-(4'-(4-Hydroxycarbamoyl-butoxy)-3'-{[(quinolin-7-ylmethyl)-amino]-methyl}-biphenyl-4-yl)-acrylic acid (35)
3-(4'-(4-Hydroxycarbamoyl-butoxy)-3'-{[(8-hydroxy-5-nitro-quinolin-7-ylmethyl)-amino]-methyl}-biphenyl-4-yl)-acrylic acid (36)
9-(3-{4-[3-Adamantan-1-yl-4'-(2-carboxy-vinyl)-biphenyl-4-yloxy]-butoxy}-2,6,6-trimethyl-cyclohex-1-enyl)-3,7-dimethyl-nona-2,4,6,8-tetraenoic acid (37).

Formulae of the above mentioned compounds are represented in the following table:

| Compound | |
|---|---|
| **1** AB 461 | 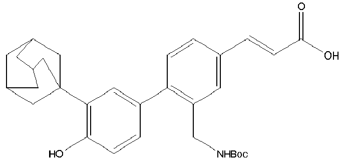 |
| **2** RC 1120 | 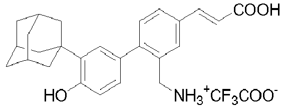 |
| **3** RC 1151 | 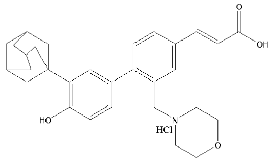 |
| **4** RC 1152 | 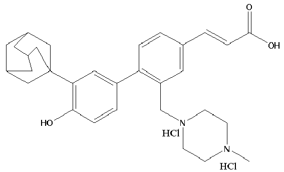 |
| **5** RC 1193 | 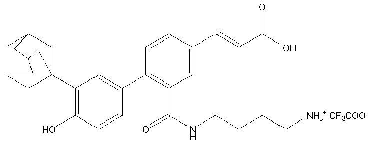 |
| **6** RC 1190 | 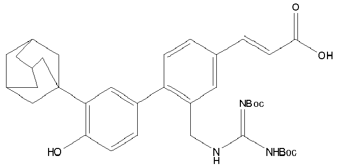 |
| **7** RC 1191 | 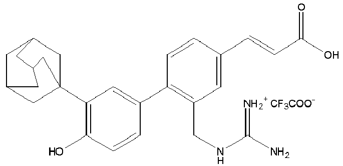 |
| **8** AB 472 | 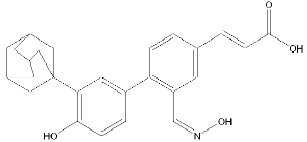 |
| **9** AB 473 | 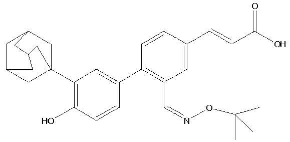 |
| **10** AB 474 | 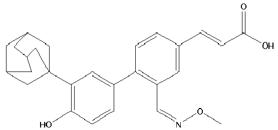 |
| **11** RC 1189 | 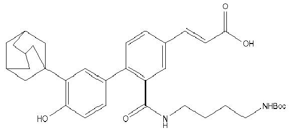 |
| **12** RC1171 | 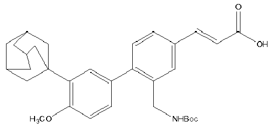 |
| **13** RC 1174 | 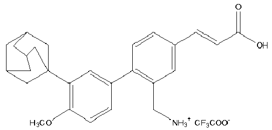 |
| **14** AB 486 | 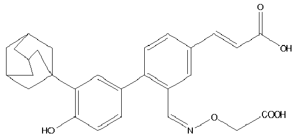 |
| **15** RC 1237 | 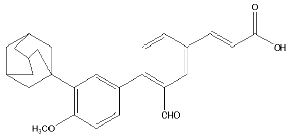 |
| **16** RC 1230 | 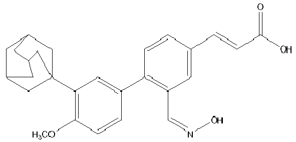 |
| **17** RC 1243 | 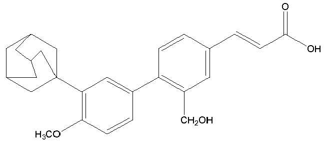 |
| **18** RC 1236 | 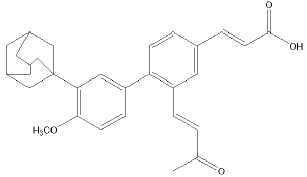 |
| **19** RC 1238 | 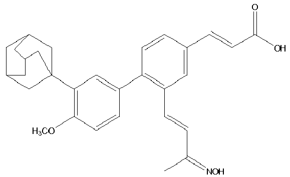 |
| **20** RC 1277 | 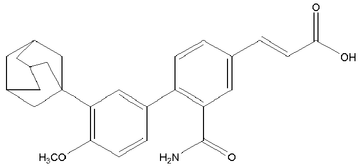 |
| **21** RC1315 | 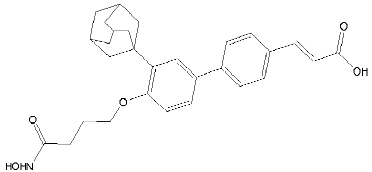 |
| **22** RC 1375 | 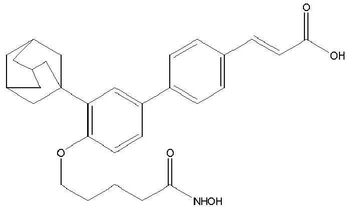 |
| **23** RC 1268 | 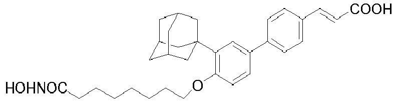 |
| **24** RC 1363 | 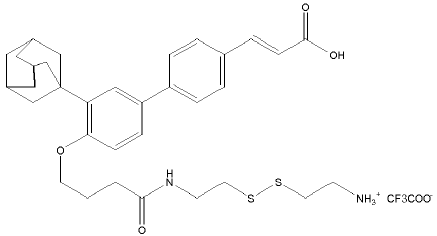 |
| **25** RC 1338 | 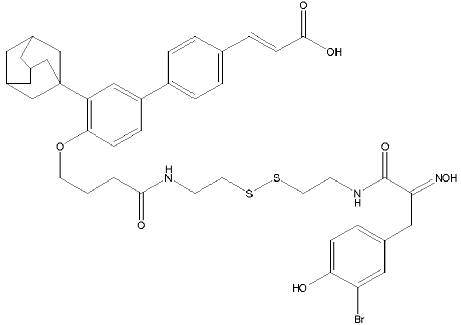 |
| **26** AB 514 | 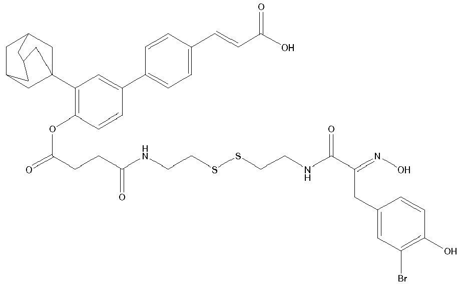 |
| **27** RC 1207 | 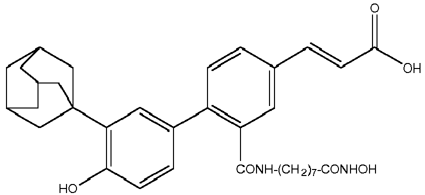 |
| **28** | 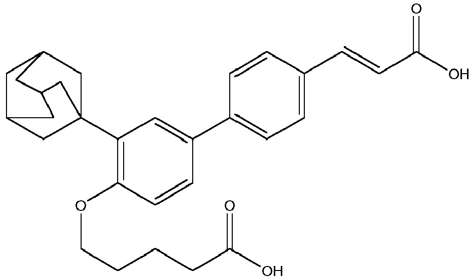 |
| **29** MIR002 | 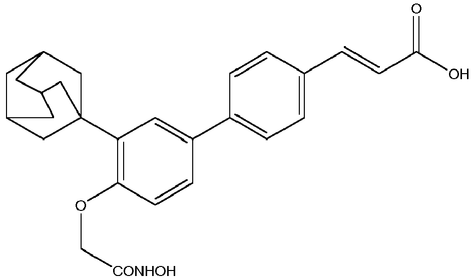 |
| **30** | 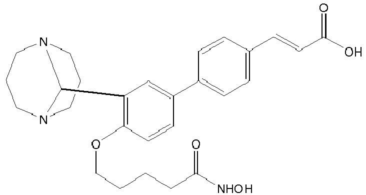 |
| **31** | 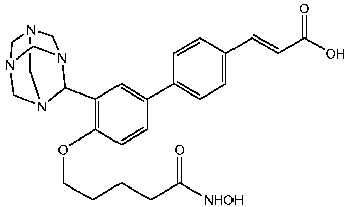 |
| **32** | 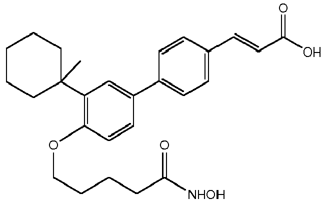 |
| **33** | 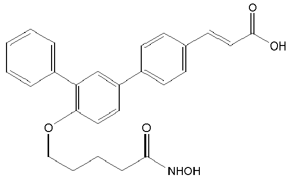 |
| **34** | 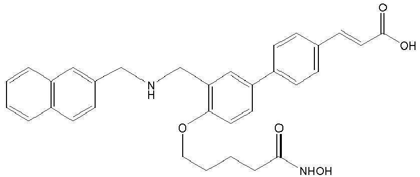 |
| **35** | 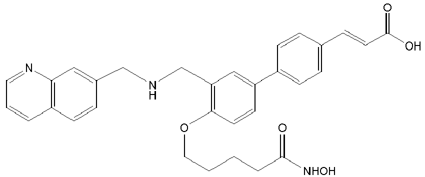 |
| **36** | 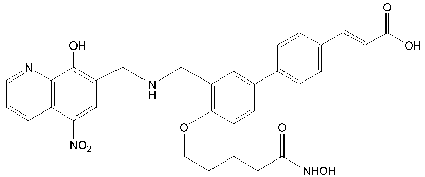 |
| **37** | 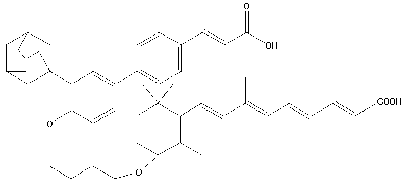 |

A process for the preparation of the compound of formula (I) is also within the scope of the present invention.

The compounds of the present invention can be synthesized by many methods available to those skilled in the art of organic chemistry. General synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared by methods described in the general schemes are given in the intermediates and examples section set out hereinafter.

The compounds of the present invention can be prepared in a number of ways known to one skilled in the art of organic synthesis. The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006)).

Compounds of formula (I) where R₂ is H, R₃ is different from H and R₅ is 1-adamantyl can be obtained for example with a Suzuki-Miyaura reaction (Miyaura, N.; Suzuki, A. Chem. Rev. (1995), 95, 2457; Mora, M. et al. Curr. Org. Chemistry (2012),16,1128-1150; Heravi, M. et al. Tetrahedron (2012), 68, 9145-9178; Alonso, F. et al. Tetrahedron (2008), 64, 3047-3101) between adamantan-1-yl-4-(4,4,5,5-tetramethyl-[1,3,2]dioxaborolan-2-yl)-phenol (A) (Cincinelli, R. et al. E. J. Med. Chem. (2014), 79, 251-259) (see Example 1, step 6), or an ether (Tribulovich, V. G. et al., Russian J. Gen. Chem. 80(4), 868-869; 2010) or a silylderivative thereof, with the triflate of a 3-substituted-4-hydroxycinnamic ester (B) where R₃ has the meaning above defined and R₄ is O-alkyl, in presence of Pd-tetrakis(triphenylphosphine) and 2M sodium or potassium carbonate, or dichloro(diphenylphosphinoferrocene)palladium.dichloromethane and potassium acetate, or a similar Pd catalyst with a similar base, in a solvent such as dimethoxyethane and ethanol, at temperatures from 25°C to 80°C (scheme 1).

Alternatively, O-substituted adamantan-1-yl-4-(4,4,5,5-tetramethyl-[1,3,2]dioxaborolan-2-yl)-phenols can be prepared from the corresponding halide and bis(pinacolato)diboron in presence of PdCl₂(dppf) and KOAc. (Giroux, A. et al. Tetrahedron Letters, (1997), 41, 3841-3844).

Alternatively, the corresponding boronic acids can be prepared by metal-halogen exchange of the corresponding halide, quenching with a trialkoxyborate reagent, and aqueous workup to provide the boronic acids (Miyaura, N. et al., Chem. Rev., (1995),95, 2457).

The esters (C) thus obtained can be hydrolyzed for example with LiOH.H₂O in THF/H₂O to obtain the corresponding acids or reacted with suitable reagents (e.g. NH₂OH or O-alkylhydroxylamines or amines) to give compounds containing the desired COR₄ group wherein R₄ has the meaning defined above, following well known procedures of organic synthesis.

The protecting group (e.g. tertbutoxycarbonyl), if present, can be eliminated by the common procedures known in the art. For example, it can be eliminated by treatment with trifluoroacetic acid in dichloromethane at T from 0°C to room T.

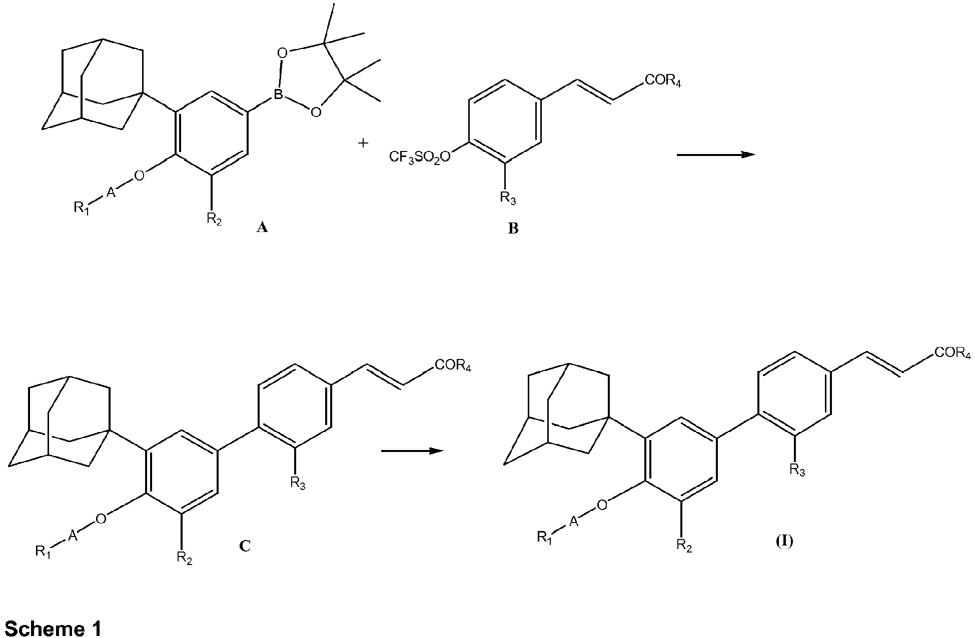

The same reaction conditions hold for compounds of structure A of scheme 1 where R₅ is alkyl, cycloalkyl, azacycloalkyl, oxacycloalkyl, aryl, heteroaryl.

Compounds of general formula A where R₂ is different from H can be obtained for example by alkylation (Cincinelli et al. Bioorg. Med. Chem. 2007, 15, 4863) of compounds D (Cincinelli R. et al. J. Med. Chem. 2005, 48, 4931-4946), followed by reaction with bis(pinacolato)diboron in presence of PdCl₂(dppf) and KOAc. (Giroux, A. et al. Tetrahedron Letters, (1997), 41, 3841-3844) (scheme 2).

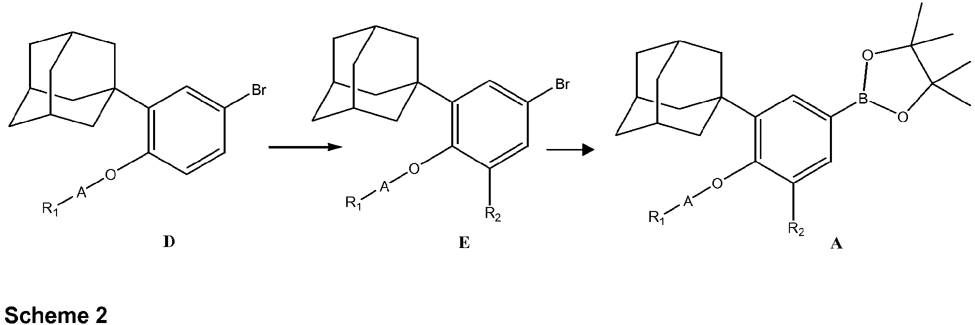

Compounds of general formula B can be obtained for example by formylation (Bhatt, S. et al. Tetrahedron Letters, 50(42), 5823-5826; 2009) or amidoalkylation (Cincinelli et al. Bioorg. Med. Chem. 2007, 15, 4863) or with other well known methods of organic synthesis, of commercially available p-bromophenol, followed by Heck coupling with a suitable acrylate (Heck, R.F. Organic Reactions, Vol. 27, 1982). The obtained 3-substituted-4-hydroxycinnamic esters can be converted into the corresponding triflates (B) by standard methods (e.g. Cincinelli, R.et al Eur. J. Med. Chem., 79, 251-259; 2014; Dallavalle, S. et al. J. Med. Chem. (2005), 48, 4931-4946) (route a, scheme 3).

Alternatively, compounds of general formula B where R₃ is for example heterocycloalkyl can be obtained by Mannich reaction of suitable 4-hydroxycinnamates with paraformaldehyde and amines (Nakano, H. J. Med. Chem., (2012), 55, 5151-5164), followed by conversion into the corresponding triflates (B) by standard methods (route b, scheme 3).

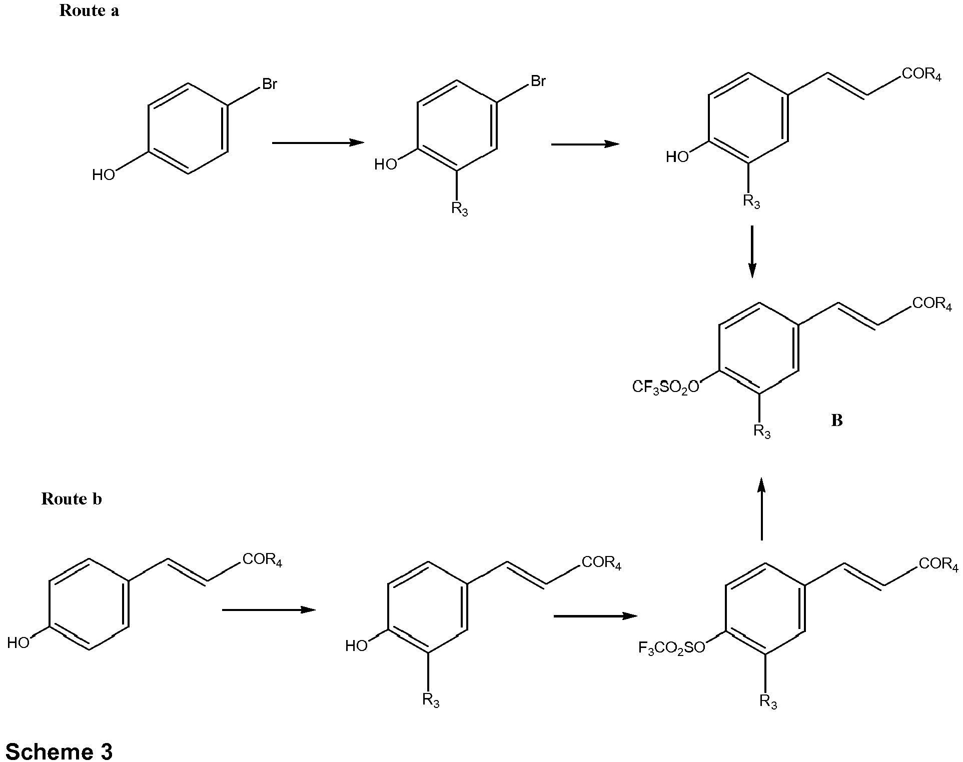

Compounds of formula (I) where R₃ is a formyl group can be converted into the corresponding oximes or alkyloximes (see examples 8-10, 16) (Cullen, M. et al. J. Med. Chem. (2007), 50, 4854-4867), alcohols (see example 17) (Kostikov, A. P. et al. J. Org. Chem. (2007), 72, 9190-9194), amides (see examples 5, 20, 27) (Xu, J. E. J. Org. Chem. (2004), 15, 3244-3253), alkenes (see examples 18-19) according to standard methods of organic synthesis.

This reaction scheme can be applied not only to esters, but also to other compounds where R₄ is different from O-alkyl.

Compounds of general formula (I) can be converted into other compounds of general formula (I) via suitable transformations. For example, compounds of formula C or (I), where R₁ is OH, can be converted into compounds where R₁ has the meanings above defined by reactions of alkylation, followed by further transformations according to general methods of organic synthesis.

Compounds of formula (I) where R₂ and R₃ are H, can be obtained by conversion of acid F (Cincinelli R. et al. J. Med. Chem. 2005, 48, 4931-4946) into a suitable ester G (for example, a tert-butyl ester, Giannini, G. et al. Bioorg. Med. Chem. (2012), 20(7),2405-2415), followed by alkylation with alkyl bromoalkylcarboxylates (Naik, Ravi et al. J. Med. Chem. (2012), 55, 10564-10571) to obtain the O-substituted intermediates H. The esters (H) thus obtained can be hydrolyzed with LiOH.H₂O in THF/H₂O to obtain the corresponding acids or reacted with suitable reagents (e.g. NH₂OH or O-alkylhydroxylamines or amines) to give compounds containing the desired COR₄ group wherein R₄ has the meaning defined above. (Scheme 4).

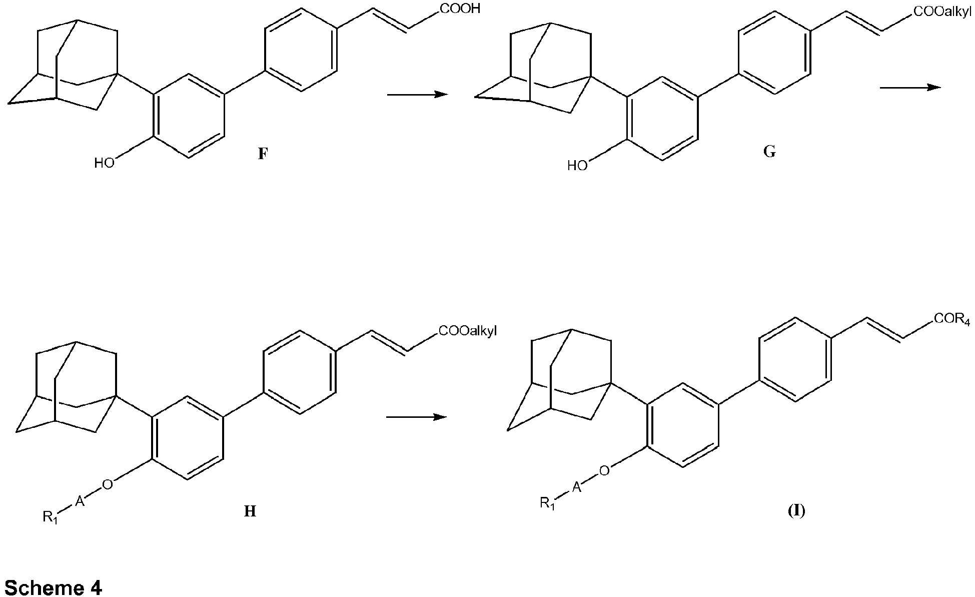

Compounds of formula (I) where R₂ and R₃ are H and R₅ is alkylaminomethyl, arylakylaminomethyl, heteroarylalkylaminomethyl can be prepared starting from the esters L (Dallavalle S. et al. Eur. J. Med. Chem. (2009), 44(5),1900-1912) via a Mannich reaction with formaldehyde and amines BNH₂, where B is alkyl, arylakyl, or heteroarylalkyl, optionally substituted with OH, Oalkyl, halogen, NO₂ and/or NH₂ depending on the desired R₅, as described for example by Sosič et al. J. Med. Chem. 2013, 56, 521-533. (Scheme 5)

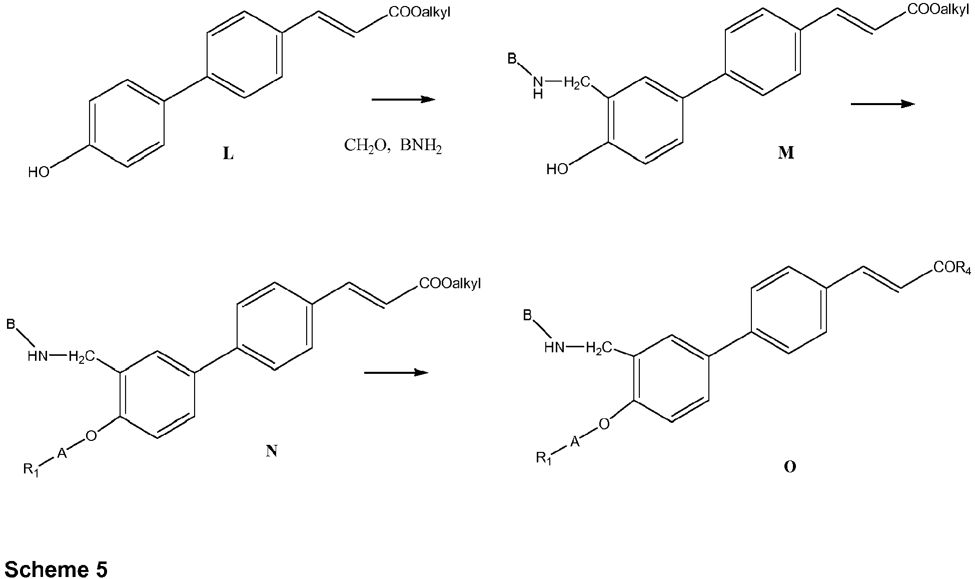

These esters can be transformed into esters N, then into compounds O following the procedures described in Scheme 4. In these transformations, the NH group of BNHCH₂ may be protected and deprotected according to the current procedures of organic synthesis.

In all the transformations above mentioned, every possibly interfering group can be protected and later deprotected according to standard procedures of organic chemistry, as described e.g. in P.G.M. Wuts and T.W. Greene "Greene's Protective Groups in Organic Synthesis", J. Wiley & Sons, Inc., 4rd Ed., 2006.

All the transformations above described are only exemplary synthetic procedures already known in organic chemistry and well known to the experts in the field.

It has been found that compounds of formula (I) are endowed with anti-proliferative activity and they are able to reduce tumor mass. Therefore, they can be advantageously used in the treatment of many cancer diseases.

It has also been found that the compounds of the invention can have an anti-tumor activity also on tumor resistant to known chemotherapy drugs, such as adarotene.

Furthermore, in many tumors the overexpression of P-glycoprotein (PGP) drastically reduces the possibility, after oral administration, of absorption of several chemotherapeutic agents (i.e. Paclitaxel, doxorubicin etc.). It has been found that the compounds of the invention are not PGP substrates, therefore they can be advantageously administered to the patients via oral route.

A further object of the present invention relates to the use of a compound of formula (I) as a medicament, in particular for the treatment of tumours. When it is used for the treatment of tumors, the antitumoral activity can be of cytotoxic nature and/or apoptotic nature, and/or antiangiogenic nature.

In a preferred embodiment, the tumour is selected from the group consisting of sarcoma, carcinoma, carcinoid, mesothelioma, lymphoma, bone tumour, neuroendocrine tumour, lymphoid leukaemia, myeloid leukaemia, monocytic leukaemia, megakaryocytic leukaemia, acute promyelocytic leukaemia, Hodgkin's disease, lung tumour, hepatoma, mesothelioma and intracranial tumor, such as glioma.

A further object of the present invention is the use of a compound of formula (I) for the prevention and treatment of tumour metastasis.

In a further embodiment of the invention, the compound of formula (I) can be used for the treatment of a disease related to altered angiogenesis.

The skilled in the art, for example a physician, can identify and recognize if a disease is related to altered angiogenesis on the basis of its general knowledge in the field.

In particular, the compound of formula (I) can be used for the treatment of the following diseases: arthritic pathology, diabetic retinopathy, psoriasis and chronic inflammatory disease.

Another object of the present invention is a pharmaceutical composition containing at least one compound of formula (I) as an active ingredient, in an amount such as to produce a significant therapeutic effect, together with pharmaceutically acceptable vehicle and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical compositions according to the invention can also contain one or more further active ingredients, in particular chemotherapeutic agents. Such agents can be any molecule which is commonly used in the treatment of tumors. Particularly preferred is a composition comprising a compound of formula (I) and a platinum-based chemotherapeutic agent, in particular cisplatin. Indeed, it has been found that such combination has a synergistic anti-tumoral activity.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (I) with suitable excipients, stabilizers and/or pharmaceutically acceptable diluents.

The following examples further illustrate the invention.

### EXAMPLES

### EXAMPLE 1

### 3-[3'-adamantan-1-yl-2-(3-tertbutoxyaminomethyl)-4'-hydroxy-biphenyl-4-yl)-acrylic acid. (1) (AB 461)

Step 1. **N-(5-Bromo-2-hydroxy-benzyl)-2-chloroacetamide.** 4-Bromophenol (1 g, 5.78 mmol) and N-hydroxymethyl-chloroacetamide (0.73 g, 5.78 mmol) in a mixture 9:1 of acetic acid and sulfuric acid are reacted at room T for 72 hours, then, after adding 290 mg of N-hydroxymethyl-chloroacetamide, again for 24 hours. Adding iced water, neutralizing with Na₂CO₃, extracting with dichloromethane, and chromatography of the crude product (898 mg) on silica gel with hexane/ethyl acetate 3:1 gives 585 mg, m.p. 159°C, ¹H-NMR (300 MHz, DMSO_{*d*6}) δ: 9.99 (brs, 1H), 8.60 (t, J= 5.72 Hz, 1H), 7.25-7.15 (m, 2H), 6.74 8d, J= 8.66 Hz, 1H), 4.18 (d, J= 5.72 Hz, 2H), 4.11 (s, 2H).
Step 2. **2-aminomethyl-4-bromophenol hydrochloride.** A mixture of N-(5-Bromo-2-hydroxybenzyl)-2-chloroacetamide (1.26 g, 4.5 mmol) in 25 ml of conc. HCl and 100 ml of ethanol for 6 hours. Concentration, azeotropic removal of water with three additions of 25 ml of ethanol, grinding the residue with ether gives a solid product, m.p. 242°C (954 mg, 88%), ¹H-NMR (300 MHz, DMSO_{*d*6}) δ: 10.52 (brs, 1H), 8.16 (brs, 3H), 7.50 (d, J= 3.36 hz, 1H), 7.37 (dd, J= 3.36 Hz, J= 8.85 Hz, 1 H), 6.90 (d, J= 8.85 Hz, 1 H), 3.92 (brs, 2H)
Step 3. **Tert-butylester of 5-bromo-4-hydroxybenzylcarbamic acid.** 2-aminomethyl-4-bromophenol hydrochloride (931 mg, 3.9 mmol), BOC-anhydride (936 mg, 4.2 mmol) e 1.08 ml (7.8 mmol) of triethylamine are dissolved in 10 ml of DMF and extracted repeatedly with dichloromethane. Drying with Na₂SO₄, evaporation and chromatography with hexane/ethyl acetate 9:1 gives 1.44 g (64%) of a white product, m.p. 127°C, ¹H-NMR (300 MHz, CDCl₃) δ: 9.12 (brs, 1 H), 7.30 (dd, J= 8.85 Hz, J= 2.44 hz, 1 H), 7.19 (d, J= 2.44 Hz, 1 H), 6.84 (d, J= 8.85 Hz, 1 H), 5.25 (brs, 1 H), 4.19 (d, J= 6.41 Hz, 2H), 1.46 (s, 9H).
Step 4. **Methyl 3-[3-(tert-butoxycarbonylamino-methyl)-4-hydroxy-phenyl]-acrylate.** A mixture of 1.14 g (3.77 mmol) of tert-butylester of 5-bromo-4-hydroxybenzylcarbamic acid, 0,52 g (6 mmol) of methyl acrylate, 17 mg (0.075 mmol) of Pd acetate and 95 mg (0.3 mmol) of tri(o-tolyl)phosphine in 1.5 ml of triethylamine is heated at 100° for 4 hours. Addition of HCl 2N to acid pH, extraction with ethyl acetate, drying over Na₂SO₄ evaporation, and chromatography through silica gel with hexane:ethyl acetate 4:2 gave 968 mg (83%) of a white product, m.p. 120°C, ¹H-NMR (300 MHz, CDCl₃) δ: 9.48 (brs, 1 H), 7.62 (d, J= 15.87 Hz, 1 H), 7.42 (dd, J= 8.54 Hz, 2.44 Hz, 1 H), 7.25 (d, J= 2.44 Hz, 1 H), 6.96 (d, J= 8.54 Hz, 1 H), 6.29 (d, J= 15.87 Hz, 1 H), 5.33 (brs, 1 H), 4.25 (d, J= 6.71 Hz, 2H), 3.81 (s, 3H).
Step 5. **Methyl 3-[3-(tert-butoxycarbonylamino-methyl)-4-trifluoromethanesulfonyloxy-phenyl]-acrylate.** A solution of 252 mg (6 mmol) of methyl 3-[3-(tert-butoxycarbonylaminomethyl)-4-hydroxy-phenyl]-acrylate in 5 ml of dry dichloromethane is added with with 0.34 ml of triethylamine, cooled at 0°C, added with 382 mg (1.07 mmol) of N-phenyl-bis-trifluoromethanesulfonimide, and left at 0°C for 2.5 hours. Adding again 59 mg (0.16 mmol) of N-phenyl-bis-trifluoromethanesulfonimide, leaving 1 hour, evaporation, addition of ethyl acetate, washing with water, drying, evaporation, and chromatography with hexane:ethyl acetate 4:1 gave 342 mg (95%) of product, m.p. 82°C.
Step 6. **2-Adamantan-1-yl-4-(4,4,5,5-tetramethyl-[1,3,2]dioxaborolan-2-yl)-phenol.** A mixture of 2-adamantanyl-4-bromophenol (3.69 g, 12.1 mmol), diboro-bis-pinacolate (3.42 g, 13.2 mmol), potassium acetate (3.53 g, 36 mmol), dichloro(diphenylphosphinoferrocene)palladium.dichloromethane (0.27 g, 0.36 mmol), in dioxane (90 mL) is heated at 80 °C in the dark for 2 hours. Evaporation of the solvent, taking up with ethyl acetate, washing with water, drying, evaporation and chromatography with hexane:ethyl acetate 9:1 gave 3.02 g (71%) of a yellowish solid, m.p. 218°C, ¹H-NMR (300 MHz, CDCl₃) δ: 7.70 (d, J= 1.53 Hz, 1 H), 7.55 (dd, J= 7.93 Hz, J= 1.53 Hz, 1 H), 6.66 (d, J= 7.93 Hz, 1 H), 2.20-2.04 (m, 9H), 1.79 (s, 6H), 1.34 (s, 12H).
Step 7. **Methyl 3-[3'-adamantan-1-yl-2-(3-tertbutoxyaminomethyl)-4'-hydroxy-biphenyl-4-yl)-acrylate** To 48 ml of a mixture 9:1 of dimethoxyethane:ethanol are added under nitrogen atmosphere with adamantan-1-yl-4-(4,4,5,5-tetramethyl-[1,3,2]dioxaborolan-2-yl)-phenol (1.46 g, 4.11 mmol), methyl 3-[3-(tert-butoxycarbonylamino-methyl)-4-trifluoromethanesulfonyloxy-phenyl]-acrylate (1.43 g, 3.26 mmol), Pd-tetrakis-triphenylphosphine (0.56 g, 0.49 mmol), and 3.26 ml of sodium carbonate 2M. Heating at 80°C for 5 hours, evaporation, taking up with ethyl acetate, washing with water, drying, evaporation, and chromatography with hexane:ethyl acetate 4:1 gave 1.0 g (59%) of product, m.p. 102°C, ¹H-NMR (300 MHz, CDCl₃) δ: 7.74 (d, J= 15.56 Hz, 1 H), 7.58 (d, J= 1.53 Hz, 1H), 7.47 (dd, J= 7.93 Hz, J= 1.53 Hz, 1H), 7.31-7.23 (m, 2H), 7.13 (d, J= 1.83 Hz, 1 H), 7.00 (dd, J= 8.24 Hz, J= 1.83 Hz, 1 H), 6.73 (d, J= 8.24 Hz, 1 H), 6.48 (d, J= 15.56 Hz, 1 H), 5.36 (brs, 1 H), 4.70 (brs, 1 H), 4.31 (d, J= 5.19 Hz, 2H), 3.84 (s, 3H), 2.19-2.08 (m, 9H), 1.79 (s, 6H), 1.47 8s, 9H).
Step 8. **3-[3'-adamantan-1-yl-2-(3-tertbutoxyaminomethyl)-4'-hydroxy-biphenyl-4-yl)-acrylic acid. (1)** A solution of 392 mg (9.35 mmol) of Li.OH.H₂O in 60 ml of a mixture 1:1 of tetrahydrofuran and water is added with with 966 mg (1.87 mmol) of methyl 3-[3'-adamantan-1-yl-2-(3-tertbutoxyaminomethyl)-4'-hydroxy-biphenyl-4-yl)-acrylate and left 20 hours at room T in the dark. Concentration in vacuo, adding ethyl ether, then HCl 2N to pH 2, separation of the phases, drying and evaporation give 828 mg of a grey solid, which is ground with dichloromethane to give a white solid, m.p. 184°C (dec)
   ¹H-NMR (300 MHz, DMSO_{*d*6}) δ: 12.4 (brs, 1H), 9.48 (s, 1H), 7.65-7.52 (m, 3H), 7.34 (t, J= 5.19 Hz, 1 H), 7.22 (d, J= 7.93 Hz, 1 H), 7.05-6.96 (m, 2H), 6.84 (d, J= 7.93 Hz, 1 H), 6.47 (d, J= 16.48 Hz, 1H), 4.11 (d, J= 5.19 Hz, 2H), 2.17-1.96 (m, 9H), 1.39 (s, 9H).

### EXAMPLE 2

### 3-(3'-Adamantan-1-yl-2-aminomethyl-4'-hydroxy-biphenyl-4-yl)-acrylic acid trifluoroacetate (2)

An ice-cooled solution of 680 mg of 3-[3'-adamantan-1-yl-2-(3-tertbutoxyaminomethyl)-4'-hydroxybiphenyl-4-y)-acrylic acid (1) in 5 ml of dichloromethane are added with dropwise 13.5 ml of trifluoroacetic acid. After 1 hour at 0°C, evaporation and drying in vacuo give 600 mg (100%) of product, m.p. 213°C, ¹H-NMR (300 MHz, DMSO_{*d*6}) δ: 9.64 (brs, 1H), 8.19 (brs, 3H), 7.95 (s, 1H), 7.68 (d, J= 8.24 Hz, 1H), 7.62 (d, J= 15.87 Hz, 1H), 7.34 (d, J= 8.24 Hz, 1H), 7.07-6.98 (m, 2H), 6.88 (d, J= 8.24 hz, 1H), 6.59 (d, J= 15.87 Hz, 1H), 4.11-4.00 (m, 2H), 2.24-1.95 (m, 9H), 1.73 (s, 6H).

### EXAMPLE 3

### 3-(3'-Adamantan-1-yl-4'-hydroxy-2-morpholin-4-ylmethyl-biphenyl-4-yl)-acrylic acid. (3)

Step 1. **Methyl 3-(4-hydroxy-3-morpholin-4-yl-methylphenyl)-acrylate** A suspension of 151 mg of paraformaldehyde (1.68 mmol) in 6.7 ml of toluene is added with with 146 mg (1,68 mmol) of morpholine and refluxed 15 minutes. 200 mg (1.12 mmol) of methyl 4-hydroxycinnamate are added with and the mixture is refluxed 5 hours. Dilution with ethyl acetate, washing with satd. NaCl and with water, drying, evaporation and chromatography with hexane:ethyl acetate 7:3 give 250 mg (80%) of a white solid, m.p. 118°C, ¹H-NMR (300 MHz, CDCl₃) δ: 7.61(d, J= 16.17 Hz, 1H), 7.40 (dd, J= 8.54 Hz, J= 2.14 Hz, 1H), 7.20 (d, J= 2.14 Hz, 1H), 6.84 (d, J= 8.54 Hz, 1H), 6.28 (d, J= 16.17 Hz, 1 H), 3.83-3.72 (m, 7H), 2.68-2.54 (m, 4H).
Step 2. **Methyl 3-(4-trifluoromethanesulfonyloxy-3-morpholin-4-yi-methylphenyl)-acrylate .** A solution of methyl 3-(4-hydroxy-3-morpholin-4-yl-methylphenyl)-acrylate (226 mg, 0.81 mmol) in 5 ml of dry dichloromethane is added with 10 mg (0.081 mmol) of dimethylaminopyridine, 0.4 ml (2.93 mmol) of triethylamine and cooled at 0°C. Further 436 mg (1.22 mmol) of N-phenyl-bis-trifluoromethanesulfonimide are added with and the mixture left at 0°C for 2,5 hours. Evaporation and chromatography with hexane:ethyl acetate 1:1 give 330 mg of product, m.p. 108 °C, H-NMR (300 MHz, CDCl₃) δ: 7.66 (d, J= 16.17 Hz, 1H), 7.62 (d, J= 2.44 Hz, 1H), 7.51 (dd, J= 8.24 Hz, J= 2.44 Hz, 1H), 7.31-7.24 (M,1H), 6.45 (d, J= 16.17 Hz, 1H), 3.82 (s, 3H), 3.74-3.68 (m, 4H), 3.55 (s, 2H), 2.50-2.43 (m, 4H).
Step 3. **Methyl 3-(3'-adamantan-1-yl-4'-hydroxy-2-morpholin-4-ylmethyl-biphenyl-4-yl)-acrylate.** A mixture of methyl 3-(4-trifluoromethanesulfonyloxy-3-morpholin-4-yl-methylphenyl)-acrylate (323 mg, 0.79 mmol), 2-adamantan-1-yl-4-(4,4,5,5-tetramethyl-[1,3,2]dioxaborolan-2-yl)-phenol (352 mg, 0.99 mmol), Na₂CO₃ 2M (0.79 ml, 1.58 mmol) and Pd (PPh₃)₄ in 12 ml of dimethoxyethane:ethanol 9:1 is heated at 80°C, under nitrogen in the dark, for 10 hours. Evaporation, taking up with ethyl acetate, washing with water, drying, evaporation and with ethyl ether:hexane 70:30 give 93 mg of a white solid, m.p. 104°C, ¹H-NMR (300 MHz, CDCl₃) δ: 7.74 (d, J= 16.17 Hz, 1H), 7.65 (s, 1H), 7.46 (d, J= 8.54 Hz, 1H), 7.32-7.24 (m, 2H), 7.22 (d,J= 1.53 Hz, 1 H), 7.09 (dd, J= 7.93 Hz, J= 1.53 Hz, 1 H), 6.69 (d, J= 8.54 Hz, 1 H), 6.48 (d, J= 16.17 Hz, 1 H), 4.92 (brs, 1H), 3.82 (s, 3H), 3.71-3.64 (m,4H), 3.41 (brs, 2H), 2.44-2.34 (m, 4H), 2.17-2.06 (m, 9H), 1.78 (s, 6H).
Step 4. **3-(3'-Adamantan-1-yl-4'-hydroxy-2-morpholin-4-ylmethyl-biphenyl-4-yl)-acrylic acid hydrochloride.** A solution of 26 mg (0.615 mmol) of Li.OH.H₂O in 4 ml of a mixture 1:1 of tetrahydrofuran and water is added with with 60 mg (0.123 mmol) of methyl 3-(3'-adamantan-1-yl-4'-hydroxy-2-morpholin-4-ylmethyl-biphenyl-4-l)-acrylate and left 20 hours at room T in the dark. Concentration in vacuo, addition of HCl 2N to pH 2 and filtration give 53 mg of a white solid, m.p. 195°C, ¹H-NMR (300 MHz, MeOD) δ: 7.91 (s, 1H), 7.75-7.61 (m, 2H), 7.38 (d, J= 7.63 Hz, 1H), 7.04-6.90 (m, 2H), 6.82 (d, J= 7.63 Hz, 1 H), 6.56 (d, J= 15.87 Hz, 1 H), 4.42 (s, 2H), 3.85-3.52 (m, 4H), 3.15-2.60 (m, 4H), 2.21-1.93 (m, 9H), 1.76 (s, 6H).

### EXAMPLE 4

### 3-(3'-Adamantan-1-yl-4'-hydroxy-3-(4-methyl-piperazin-1-ylmethyl)-biphenyl-4-yl)-acrylic acid (4)

Step 1. **Methyl 3-[4-Hydroxy-3-(4-methyl-piperazin-1-ylmethyl)-phenyl]-acrylate.** A suspension of 151 mg (1.68 mmol) of paraformaldehyde in 6.7 ml of toluene is added with with 168 mg (1.68 mmol) of N-methylpiperazine and refluxed 10 minute, then added with with 200 mg (1.12 mmol) of methyl 4-hydroxycinnamate and refluxed for 5 hours. Dilution with ethyl acetate, washing with sad. NaCl, then with water, drying, evaporation and chromatography with hexane:ethyl acetate 7:3 gives 234 mg (72%) of a white solid, m.p. 93°C, H-NMR (300 MHz, CDCl₃) δ: 7.61 (d, J= 16.17 Hz, 1 H), 7.38 (dd, J= 8.54 Hz, J= 1.83 Hz, 1 H), 7.19 (d, J= 1.83 Hz, 1 H), 6.83 (d, J= 8.54 Hz, 1 H), 6.27 (d, J= 16.17 Hz, 1H), 3.80 (s, 3H), 3.75 (s, 2H), 2.90-2.38 (m, 8H), 2.32 (s, 3H).
Step 2. **Methyl 3-[4-trifluoromethanesulfonyloxy-3-(4-methyl-piperazin-1-ylmethyl)-phenyl]acrylate.** A solution of methyl 3-[4-hydroxy-3-(4-methyl-piperazin-1-ylmethyl)-phenyl]-acrylate (205 mg, 0.71 mmol) in 5 ml of dry dichloromethane is added with 10 mg (0.081 mmol) of dimethylaminopyridine, 0.4 ml (2.93 mmol) of triethylamine, cooled at 0°C, then added with 328 mg (0.91 mmol) of N-phenyl-bis-trifluoromethanesulfonimide and left at room T for 3 hours. Evaporation and chromatography with dichloromethane:methanol 85:15 give 281 mg (94%) of a white solid, m.p. 93.5°C. ¹H-NMR (300 MHz, CDCl₃) δ: 7.68 (d, J= 16.17 Hz, 1H), 7.62 (d, J= 2.14 Hz, 1H), 7.52 (dd, J= 8.54 Hz, J= 2.14 Hz, 1H), 7.31-7.26 (m, 1H), 6.47 (d, J= 16.17 Hz, 1H), 3.84 (s, 3H), 3.58 (s, 2H), 2.64-2.47 (m, 4H), 2.11-1.92 (m, 4H).
Step 3. **Methyl 3-(3'-adamantan-1-yl-4'-hydroxy-3-(4-methyl-piperazin-1-ylmethyl)-biphenyl-4-yl)-acrylate.** A mixture of methyl 3-[4-trifluoromethanesulfonyloxy-3-[(4-methyl-piperazin-1-ylmethyl)-phenyl]acrylate (260 mg, 0.62 mmol), 2-adamantan-1-yl-4-(4,4,5,5-tetramethyl-[1,3,2]dioxaborolan-2-yl)-phenol (275 mg, 0.78 mmol), Na₂CO₃ 2M (0.62 ml, 1.23 mmol) and Pd(PPh₃)₄ (107 mg, 0.092 mmol) in 9 ml of dimethoxyethane:ethanol 9:1 is heated at 80°C, under nitrogen and in the dark, for 12 hours. Evaporation, taking up with ethyl acetate, washing with water, drying, evaporation and chromatography with ethyl acetate : methanol 7:1 give 84 mg of a white solid, m.p. 240 °C. ¹H-NMR (300 MHz, DMSO-_{*d*6}) δ: 9.42 (s, 1H), 7.73-7.62 (m, 3H), 7.26 (d, J= 8.24 Hz, 1 H), 7.22 (d, J= 1.22 Hz, 1 H), 7.06 (dd, J= 8.24 Hz, J= 1.22 Hz, 1 H), 6.81 (d, J= 8.24 Hz, 1H), 6.62 (d, J= 15.56 Hz, 1H), 3.73 (s, 3H), 3.30 (s, 2H), 2.40-2.21 (m, 8H), 2.16-2.00 (m, 12 H), 1.72 (s, 6H).
Step 4. **3-(3'-Adamantan-1-yl-4'-hydroxy-3-(4-methyl-piperazin-1-ylmethyl)-biphenyl-4-yl)-acrylic acid hydrochloride.** A solution of 25 mg (0.60 mmol) of Li.OH.H₂O in 5 ml of a mixture 1.5:1 of tetrahydrofuran and water is added with with 60 mg (0.120 mmol) of methyl 3-(3'-adamantan-1-yl-4'-hydroxy-3-(4-methyl-piperazin-1-ylmethyl)-biphenyl-4-yl)-acrylate and left 20 hours at room T in the dark. Concentration in vacuo, cooling with ice, adding HCl 2N to pH 2, and filtration give 55 mg (87%) of a white solid, m.p. 250°C. ¹H-NMR (300 MHz, MeOD) δ: 7.91 (s, 1 H), 7.75-7.61 (m, 2H), 7.38 (d, J= 7.63 Hz, 1 H), 7.04-6.90 (m, 2H), 6.82 (d, J= 7.63 Hz, 1 H), 6.56 (d, J= 15.87 Hz, 1H), 4.42 (s, 2H), 3.85-3.52 (m, 4H), 3.15-2.60 (m, 4H), 2.21-1.93 (m, 9H), 1.76 (s, 6H).

### EXAMPLE 5.

### 3-[3'-Adamantan-1-yl-2-(4-amino-butylcarbamoyl)-4'-hydroxy-biphenyl-4-yl]-acrylic acid trifluoroacetate (RC 1193) (5)

Step 1. **3'-Adamantan-1-yl-4'-(tert-butyl-dimethyl-silanyloxy)-4-(2-methoxycarbonyl-vinyl)-biphenyl-2-carboxylic acid.** A solution of 390 mg (0.73 mmol) of methyl 3-[3'-adamantan-1-yl-4'-(tert-butyl-dimethyl-silanyloxy)-2-formyl-biphenyl-4-yl]-acrylate (R. Cincinelli et al. Journal of Medicinal Chemistry (2005), 48, 4931-4946) in 5 ml of tert-butanol is added with 0.32 ml (3.00 mmol) of 2-methyl-2-butene, 200 mg (2.21 mmol) of NaClO₂ and a solution of 202 mg (1.41 mmol) of NaH₂PO₄ in 2 ml of water. Stirring 1.5 hours at room T, extraction with ethyl acetate, drying and evaporating the extract gives a residue that is crystallized from methylene chloride/hexane to give 330 mg of a white solid. ¹H-NMR (300 MHz, DMSO-_{d6}) δ: 7.92 (d, J = 1.5 Hz, d), 7.87 (dd, J = 7.9, 1.5 Hz, dd), 7.72 (d, J = 16.1 Hz, 1H), 7.45 (d, J = 7.9 Hz, d), 7.20-7.13 (m, 2H), 6.88 (d, J = 8.2 Hz, 1H), 6.72 (d, J = 16.1 Hz, 1H), 3.74 (s, 3H), 2.07 (s, 9H), 1.74 (s, 6H), 1.03 (s, 9H), 0.35 (s, 6H).
Step 2. **Methyl 3-[3'-adamantan-1-yl-2-(4-tert-butoxycarbonylamino-butylcarbamoyl)-4'-(tert-butyl-dimethyl-silanyloxy)-biphenyl-4-yl]-acrylate.** A suspension of 150 mg (0.27 mmol) of 3'-adamantan-1-yl-4'-(tert-butyl-dimethyl-silanyloxy)-4-(2-methoxycarbonyl-vinyl)-biphenyl-2-carboxylic in 3.2 ml of dry dichloromethane is added, under nitrogen atmosphere, with 177 mg (1.37 mmol) of diisopropylethylamine, 57 mg (0.30 mmol) of tert-butylester of 4-amino-butylcarbamic acid, and 159 mg (0.35 mmol) of benzotriazol-1-yloxy)tris(dimethylaminol)phosphonium hexafluorophosphate, and left 48 hours at room T. Evaporation, and chromatography hexane/ethyl acetate 3:1 give 140 mg of a white solid, m.p. 93°C. ¹H-NMR (300 MHz, CDCl₃) δ: 7.86 (d, J = 1.8 Hz, 1H), 7.72 (d, J = 16.2 Hz, 1H), 7.59 (dd, J = 7.9, 1.8 Hz, 1H), 7.39 (d, J = 7.9 Hz, 1H), 7.29-7.26 (m, 2H), 7.14 (dd, J = 8.2, 2.4 Hz, 1H), 6.86 (d, J = 8.2 Hz, 1H), 6.51 (d, J = 16.2 Hz, 1H), 5.39 (brs, 1H), 4.50 (brs, 1H), 3.83 (3H, s), 3.24-3.15 (m, 2H), 3.04-2.95 (m, 2H), 2.11 (s, 9H), 1.78 (s, 6H), 1.64 (s, 6H), 1.42 (s, 9H), 1.32-1.24 (m, 4H), 1.07 (s, 9H).
Step 3. **3-[3'-Adamantan-1-yl-2-(4-tert-butoxycarbonylamino-butylcarbamoyl)-4'-hydroxybiphenyl-4-yl]-acrylic acid (11)**. A solution of 113 mg (0.17 mmol) of methyl 3-[3'-adamantan-1-yl-2-(4-tert-butoxycarbonylamino-butylcarbamoyl)-4'-(tert-butyldimethylsilanyloxy)-biphenyl-4-yl]-acrylate in 6.7 ml of a 1 N solution of NaOH in methanol is refluxed for 5 hours. Evaporation, taking up with water, cooling in ice, and slow addition of HCl 2N to acid pH, filtration and drying gives a yellowish solid, 113 mg, m.p. 147°C. ¹H-NMR (300 MHz, MeOD) δ: 7.96 (brs, 1H), 7.71-7.56 (m, 3H), 7.39 (d, J = 7.9 Hz, 1H), 7.20 (d, J = 1.8 Hz, 1H), 7.08 (dd, J = 8.2, 2.1 Hz, 1H), 6.73 (d, J = 8.2 Hz, 1 H), 6.50 (d, J = 16.2 Hz, 1 H), 3.18-3.08 (m, 2H), 2.90 (t, J = 6.7 Hz, 2H), 2.19-1.96 (m, 9H), 1.77 (s, 6H), 1.33 (s, 9H, 1.35-1.13 (m, 4H).
Step 4. **3-[3'-Adamantan-1-yl-2-(4-amino-butylcarbamoyl)-4'-hydroxy-biphenyl-4-yl]-acrylic acid trifluoroacetate (5).** A solution of 68 mg (0.12 mmol) of 3-[3'-adamantan-1-yl-2-(4-tert-butoxycarbonylamino-butylcarbamoyl)-4'-hydroxy-biphenyl-4-yl]-acrylic acid in 1.2 ml of dichloromethane, is cooled to 0°C, then added with 1.2 ml of trifluoroacetic acid, and left for 4 hours. Evaporation, taking up with diethyl ether, and filtration give 59 mg of a product, m.p. 134°C. ¹H-NMR (300 MHz, MeOD) δ: 7.60 (s, 1H), 7.43 (d, J = 8.2 Hz, 1H), 7.20 (d, J = 1.5 Hz, 1H), 7.11 (dd, J = 8.2, 1.5 Hz, 1H), 6.75 (d, J = 8.2 Hz, 1H), 6.51 (d, J = 16.2 Hz, 1H), 3.21-3.14 (m, 2H), 2.90-2.80 (m, 2H), 2.20-1.97 (m, 9H), 1.79 (s, 6H), 1.53-1.39 (m, 4H).

### EXAMPLE 6

### 3-(3'-Adamantan-1-yl-2-(ditertbutoxycarbonylguanidino)methyl)-4'-hydroxy-biphenyl-4-yl)-acrylic acid (6). RC 1190

A suspension of 113 mg (0.22 mmol) of 3-(3'-adamantan-1-yl-2-aminomethyl-4'-hydroxy-biphenyl-4-yl)-acrylic acid trifluoroacetate (EXAMPLE 2) in 2.2 ml of dichloromethane is added with 60 µl (2 eq.) of triethylamine, then, after 5 minutes, added with 94 mg (0.24 mmol) of N,N'-Di-Boc-N"-trifluoromethanesulfonyl-guanidine and another eq. of triethylamine, and left under a nitrogen atmosphere and in the dark for 12 hours. Taking up with 15 ml of dichloromethane, washing with a solution 2M of NaHSO₄, washing with water, drying over Na₂SO₄, and evaporation gives 133 mg of a crude product. Crystallization from dichloromethane/methanol (5 ml : 0.25 ml) gives 30 mg of pure product, whereas chromatography of the mother liquid with dichloromethane/methanol gives further 70 mg, m.p. 280°C. ¹H-NMR (300 MHz, MeOD) δ: 7.73-7.64 (m, 2H), 7.58 (dd, J = 8.2, 1.8 Hz, 1 H), 7.31 (d, J = 8.2 Hz, 1 H), 7.04 (d, J = 2.1 Hz, 1 H), 6.96 (dd, J = 8.2, 2.1 Hz, 1 H), 6.76 (d, J = 8.2 Hz, 1H), 6.50 (d, J = 16.2 Hz, 1H), 4.31 (s, 2H), 2.15-1.94 (m, 9H), 1.77 (s, 6H), 1.46 (s, 9H), 1.42 (s, 9H).

### EXAMPLE 7

**3-(3'-Adamantan-1-yl-2-guanidinomethyl-4'-hydroxy-biphenyl-4-yl)-acrylic acid trifluoroacetate. (7)** A solution of 70 mg (0.11 mmol) of 3-(3'-adamantan-1-yl-2-(ditertbutoxycarbonylguanidino)methyl)-4'-hydroxy-biphenyl-4-yl)-acrylic acid (6) in 1.1 ml of dichloromethane is slowly added under nitrogen atmosphere, 1.1. ml of trifluoroacetic acid, and left 12 hours in the dark. Evaporation and chromatography with silica gel with CH₂Cl₂/MeOH/TFA 18:2:0.2 as an eluent gives a hygroscopic product, (67 mg, m.p. 163°C). ¹H-NMR (300 MHz, MeOD) δ: 7.69 (d, J = 16.2 Hz, 1 H), 7.64-7.58 (m, 2H), 7.32 (d, J = 8.2 Hz, 1 H), 7.08 (d, J = 1.5 Hz, 1 H), 6.97 (dd, J = 8.2, 1.5 Hz, 1 H), 6.78 (d, J = 8.2 Hz, 1 H), 6.50 (d, J = 16.2 Hz, 1 H), 4.27 (s, 2H), 2.20-1.96 (m, 9H), 1.78 (s, 6H).

### EXAMPLE 8

### 3-[3'-Adamantan-1-yl-4'-hydroxy-2-hydroxyiminomethyl-biphenyl-4-yl]-acrylic acid (8).

Step 1. **Methyl 3-[3'adamantan-1-yl-4'-(tert-butyl-dimethyl-silanyloxy)-2-hydroxyiminomethyl-biphenyl-4-yl]-acrylate.** A solution of 106 mg (0.20 mmol) of methyl 3-[3'-adamantan-1-yl-4'-(tert-butyl-dimethyl-silanyloxy)-2-formyl-biphenyl-4-yl]-acrylate (R. Cincinelli et al. Journal of Medicinal Chemistry (2005), 48, 4931-4946) in 10 ml of ethanol is added with 1.5 ml of pyridine and 45 mg (0.65 mmol) of hydroxylamine hydrochloride, and refluxed 1 hour. Evaporation and chromatography with hexane/ethyl acetate 4:1 give 100 mg of product. ¹H-NMR (300 MHz, CDCl₃) δ:8.18 (s,1H), 8.06 (s, 1H), 7.74 (d, J= 16.2 Hz, 1H), 7.58 (dd, J = 8.2, 1.8 Hz, 1H), 7.50 (brs, 1H), 7.40 (d, J = 8.2 Hz, 1H), 7.19 (d, J = 1.8 Hz, 1H), 7.03 (dd, J = 8.2, 1.8 Hz, 1H), 6.87 (d, J = 8.2 Hz, 1 H), 6.53 (d, J = 16.2 Hz, 1 H), 3.84 (s, 3H), 2.20-2.07 (m, 2H), 1.79 (s, 6H), 1.09 (s, 9H), 0.40 (s, 6H).
Step 2. **3-[3'-Adamantan-1-yl-4'-hydroxy-2-hydroxyimino-methyl-biphenyl-4-yl]-acrylic acid (8):**
   100 mg (0.18 mmol) of methyl 3-[3'-adamantan-1-yl-4'-(tert-butyl-dimethyl-silanyloxy)-2-hydroxyiminomethyl-biphenyl-4-yl]-acrylate are dissolved in 6 ml of a solution 1N of NaOH in methanol and the mixture is refluxed for 5 hours. Evaporation, taking up with water, cooling with ice, slow addition of HCl 1N to acid pH, filtration, and drying gives the product, 70 mg, m.p. 230°C. ¹H-NMR (300 MHz, DMSO-*d*₆) δ: 12.4 (brs, 1 H), 11.3 (s, 1 H), 9.62 (s, 1 H), 7.96 (d, J = 1.8 Hz, 1 H), 7.94 (s, 1 H), 7.77 (dd, J = 8.2, 1.8 Hz, 1 H), 7.64 (d, J = 16.2 Hz, 1 H), 7.37 (d, J = 8.2 Hz, 1 H), 7.04-6.96 (m, 2H), 6.87 (d, J = 8.2 Hz, 1H), 6.52 (d, J = 16.2 Hz, 1H), 2.16-1.97 (s, 9H), 1.72 (s, 6H).

### EXAMPLE 9

### 3-[3'-Adamantan-1-yl-4'hydroxy-2-tert-butoxyiminomethyl-biphenyl-4-yl]-acrylic acid. (9) (AB473)

Step 1. **Methyl 3-[3'-adamantan-1-yl-4'-(tert-butyl-dimethyl-silanyloxy)-2-tert-butoxyiminomethyl-biphenyl-4-yl]-acrylate.** A solution of 100 mg (0.19 mmol) of methyl 3-[3'-adamantan-1-yl-4'-(tert-butyl-dimethyl-silanyloxy)-2-formyl-biphenyl-4-yl]-acrylate (R. Cincinelli et al. Journal of Medicinal Chemistry(2005),48, (4931-4946) in 9 ml of ethanol is added with 1.4 ml of pyridine and 78 mg (0.62 mmol) of O-tertbutylhydroxylamine hydrochloride, and refluxed for 1 hour. Evaporation gave a crude that was used for the next step without further purification
Step 2. **3-[3'-Adamantan-1-yl-4'-hydroxy-2-tert-butoxyiminomethyl-biphenyl-4-yl]-acrylic acid. (9)**
   100 mg (0.18 mmol) of methyl 3-[3'-adamantan-1-yl-4'-(tert-butyl-dimethyl-silanyloxy)-2-tert-butoxyiminomethyl-biphenyl-4-yl]-acrylate are dissolved in 6 ml of a solution 1N of NaOH in methanol and the mixture refluxed for 5 hours. Evaporation, taking up with water, cooling with ice, slow addition of HCl 1 N to acid pH, filtration, and drying gives 65 mg of a yellowish product, m.p. 239°C. ¹H-NMR (300 MHz, CDCl₃) δ: 8.12 (1H, s); 8.06 (1H, s); 7.87 (1H, d, J = 16.2 Hz); 7.59 (1 H, dd, J = 8.2, 1.5 Hz); 7.38 (1 H, d, J = 8.2 Hz); 7.18 (1 H, d, J = 1.8 Hz); 7.05 (1 H, dd, J = 8.2, 1.8 Hz); 6.73 (1 H, d, J = 8.2 Hz); 6.54 (1 H, d, J = 16.2 Hz); 2.19-2.05 (9H, m), 1.80 (6H, s); 1.45 (9H, s).

### EXAMPLE 10

### 3-[3'-Adamantan-1-yl-4'-hydroxy-2-methoxyimino-methyl-biphenyl-4-yl]-acrylic acid. (10)

Step 1. **Methyl 3-[3'-adamantan-1-yl-4'-(tert-butyl-dimethyl-silanyloxy)-2-methoxyiminomethyl-biphenyl-4-yl]-acrylate.** A solution of 100 mg (0.19 mmol) of methyl 3-[3'-adamantan-1-yl-4'-(tert-butyl-dimethyl-silanyloxy)-2-formyl-biphenyl-4-yl]-acrylate (R. Cincinelli et al. Journal of Medicinal Chemistry (2005),48,4931-4946) in 9 ml of ethanol is added with 1.4 ml of pyridine and 52 mg (0.62 mmol) of O-methylhydroxylamine hydrochloride, and refluxed 1 hour. Evaporation gave a crude that was used for the next step without further purification.
Step 2. **3-[3'-Adamantan-1-yl-4'-hydroxy-2-methoxyimino-methyl-biphenyl-4-yl]-acrylic** acid 85 mg (0.15 mmol) of methyl 3-[3'-adamantan-1-yl-4'-(tert-butyl-dimethyl-silanyloxy)-2-methoxyimino-methyl-biphenyl-4-yl]-acrylate are dissolved in 5 ml of a solution 1 N of NaOH in methanol and the mixture refluxed for 5 hours. Evaporation, taking up with water, cooling with ice, slow addition of HCl 1 N to acid pH, filtration, and drying give a yellowish product, 55 mg, m.p. 224°C. ¹H-NMR (300 MHz, CDCl₃) δ: 8.13 (1H, s); 8.07 (1H, s); 7.85 (1H, d, J = 16.2 Hz); 7.60 (1H, dd, J = 8.2, 1.8 Hz); 7.40 (1H, d, J = 8.2 Hz); 7.18 (1H, d, J = 2.1 Hz); 7.03 (1H, dd, J = 8.2, 2.1 Hz) 6.74 (1H, d, J = 8.2 Hz); 6.55 (1H, d, J = 16.2 Hz); 4.00 (3H, s); 2.21-2.06 (9H, m), 1.80 (6H, s).

### EXAMPLE 11

### 3-[3'-Adamantan-1-yl-2-(4-tert-butoxycarbonylamino-butylcarbamoyl)-4'-hydroxy-biphenyl-4-yl]-acrylic acid (11)

The compound was prepared as described in Example 5, Step 3.

### EXAMPLE 12

### 3-[3'-Adamantan-1-yl-2-(3-tertbutoxyaminomethyl)-4'-methoxy-biphenyl-4-yl)-acrylic acid. (12)

Step 1. **Methyl 3-[3'-adamantan-1-yl-2-(3-tertbutoxyaminomethyl)-4'-methoxy-biphenyl-4-yl)-acrylate.** A solution of 3-[3'-adamantan-1-yl-2-(3-tertbutoxyaminomethyl)-4'-hydroxy-biphenyl-4-yl)-acrylate (140 mg, 0.26 mmol) (EXAMPLE 1) in acetonitrile (10 ml) is added with 210 mg (1.5 mmol) of potassium carbonate and 60 µl (0.96 mmol) of methyl iodide, and refluxed for 3 h. Filtration, evaporation, and chromatography with hexane : ethyl acetate 3:1.give 122 mg of a white solid, m.p. 144 °C. ¹H-NMR (300 MHz, CDCl₃) δ: 7.74 (1H, d, J = 16.2 Hz); 7.59 (1H, d, J = 1.8 Hz); 7.48 (1 H, dd, J = 8,5, 1.8 Hz); 7.32-7.26 (1 H, m); 7.17-7.09 (2H, m); 6.93 (1 H, d, J = 8.5 Hz); 6.43 (1 H, d, J = 16.2 Hz); 4.68 (1 H, brs); 4.31 (2H, d, J = 5.2 Hz); 3.89 (3H, s); 3.84 (3H, s), 2.16-2.07 (3H, m); 1.78 (6H, s); 1.46 (9H, s).
Step 2. **3-[3'-Adamantan-1-yl-2-(3-tert-butoxyaminomethyl)-4'-methoxy-biphenyl-4-yl)-acrylic acid (12):** A solution of methyl 3-[3'-adamantan-1-yl-2-(3-tertbutoxyaminomethyl)-4'-hydroxybiphenyl-4-yl)-acrylate (100 mg, 0.19 mmol) in THF (3 ml) is added with 3 ml of water and LiOH.H₂O (39.4 mg, 0.94 mmol) and left at room T and in the dark for 48 hours. Concentration in vacuo, addition of HCl 1N to pH 2 and filtration give 97 mg of a white solid, m.p. 159°C. ¹H-NMR (300 MHz, DMSO-*d*₆) δ: 7.58 (1 H, s); 7.52 (1 H, dd, J = 8.2, 1.5 Hz); 7.44 (1 H, d, J = 16.2 Hz); 7.36 (1 H, t, J = 5.2 Hz); 7.25-7.15 (2H, m); 7.09 (1 H, d, J = 1.5 Hz); 7.04 (1 H, d, J = 8.2 Hz); 6.47 (1 H, d, J = 16.2 Hz); 4.09 (2H, d, J = 5.2 Hz); 3.84 (3H, s); 2.11-1.97 (9H, m); 1.73 (6H, s); 1.39 (9H, s).

### EXAMPLE 13

### 3-[3'-Adamantan-1-yl-2-(3-aminomethyl)-4'-methoxy-biphenyl-4-yl]-acrylic acid trifluoroacetate (13)

A suspension of 3-[3'-adamantan-1-yl-2-(3-tert-butoxyaminomethyl)-4'-methoxy-biphenyl-4-yl)-acrylic acid (77 mg, 0.149 mmol) **(12)** in dry CH₂Cl₂ (1.49 ml) cooled at 0°C is added with 1.49 ml of trifluoroacetic acid, and stirred at 0°C for 2 hours. Evaporation and crystallization from ethyl ether give 47 mg of product, m.p. 152 °C.

¹H-NMR (300 MHz, MeOD) δ: 7.75 (1H, s); 7.71 (1H, d, J = 16.2 Hz); 7.67 (1H, dd, J = 8.2, 1.5 Hz); 7.37 (1H, d, J = 8.2 Hz); 7.18-7.11 (2H, m); 7.04 (1H, d, J = 8.2 Hz); 6.56 (1H, d, J = 16.2 Hz); 4.14 (2H, s); 3.87 (3H, s); 2.15-2.00 (9H, m); 1.79 (6H, s).

### EXAMPLE 14

### 3-[3'-Adamant-1-yl-4'-hydroxy-2-carboxymethoxyimino-methyl-biphenyl-4-yl]-acrylic acid. (14)

A solution of 70 mg of methyl 3-[3'-adamantan-1-yl-4'-hydroxy-2-(formyl)-biphenyl-4-yl]-acrylic acid in 4 ml of ethanol is treated with 1 eq of pyridine, then with 20 mg of aminoxyacetic acid and left at room T for 24 hrs. Evaporation and column chromatography with CH₂Cl₂:MeOH 4:1 give 65 mg of the product, m.p. 108-110°C (dec). ¹H-NMR (300 MHz, DMSO-*d*₆) δ: 9.68 (1 H,s); 8.07 (1 H, s); 7.93 (1H, d, J= 1.53 Hz); 7.82 (1H, dd, J= 8.24 Hz, 1.53 Hz); 7.64 (1H, d, J= 16.2 Hz); 7.43 (1H, d, J= 8.24 Hz); 7.06-6.99 (2H, m); 6.88 (1 H, d, J= 8.24 Hz); 6.54 (1 H, d, J= 16.2 Hz); 4.66 (2H, s); 2.14-1.99 (9H, m); 1.72 (6H, s).

### EXAMPLE 15

### 3-[3'-Adamantan-1-yl-4'-methoxy-2-(formyl)-biphenyl-4-yl]-acrylic acid (15)

### Step 1. Methyl 3-[3'-adamantan-1-yl-4'-methoxy-2-(formyl)-biphenyl-4-yl]-acrylate

To a solution of 200 mg (0.54 mmol) of 2-adamantan-1-yl-4-(4,4,5,5-tetramethyl-[1,3,2]dioxaborolan-2-yl)-anisol in 4 ml of dry 1,4-dioxane were added 367 mg (1.08 mmol) of methyl 3-formyl-4-trifluoromethanesulfonyloxy-phenylacrylate, 0,68 ml (1.36 mmol) of 2M aq. Na₂CO₃, and 12.2 mg (0.016 mmol) of dichloro(diphenylphosphinoferrocene)palladium.dichloromethane. The mixture was heated 3 hrs in the dark and under a nitrogen atmosphere at 80-90°C, then added with water and extracted with EtOAc. The extracted layer was dried, evaporated and crystallized from AcOEt to give 156 mg of methyl 3-[3'-adamantan-1-yl-4'-methoxy-2-(formyl)-biphenyl-4-yl]-acrylate, m.p. 189°C, ¹H-NMR (300 MHz, CDCl₃) δ: 10.0 (s, 1 H), 8.16 (1 H, d, J = 1.83 Hz), 7.72-7.83 (2H, m), 7.52 (1 H, d, J = 8.2 Hz), 7.20-7.25 (2H, m), 6.99 (1 H, d, J = 8.2 Hz), 6,57 (1 H, d, J = 16.2 Hz), 3.92 (3H, s), 3.85 (3H, s), 2.05-2.16 (9H, m), 1.79 (6H, s).

### Step 2. 3-[3'-adamantan-1-yl-4'-methoxy-2-(formyl)-biphenyl-4-yl]-acrylic acid (15)

Methyl 3-[3'-adamantan-1-yl-4'-methoxy-2-(formyl)-biphenyl-4-yl]-acrylate (70 mg, 0.16 mmol) was added to a solution of LiOH.H₂O (34 mg, 0.8 mmol) in 5 ml of THF/H₂O 1:1, and the mixture left overnight at room T in the dark. Evaporation, addition of 2N HCl, and filtration gave 67 mg of the product as a white solid, m.p. 240°C, ¹H-NMR (300 MHz, CDCl₃) δ: 12.5 (1H, br s), 9.89 (1H, s), 8.01-8.11 (2H, m), 7.70 (1H, d, J = 16.2 Hz), 7.58 (1H, d, J = 8.24 Hz), 7.31 (1H, dd, J = 1.83 Hz, J = 8.24 Hz), 7.16 (1H, d, J = 1.83 Hz), 7.13 (1H, d, J = 8.24 Hz), 6.64 (1H, d, J = 16.2 Hz), 3.87 (3H, s), 1.93-2.12 (9H, m), 1.73 (6H, s).

### EXAMPLE 16

### 3-[3'-Adamantan-1-yl-4'-methoxy-2-(hydroxyiminomethyl)-biphenyl-4-yl]-acrylic acid (16)

### Step 1. 3-[3'-adamantan-1-yl-4'-methoxy-2-(hydroxyimino-methyl)-biphenyl-4-yl]-acrylate

Methyl 3-[3'-adamantan-1-yl-4'-methoxy-2-(formyl)-biphenyl-4-yl]-acrylate (87 mg, 0.20 mmol) was suspended in 1.9 ml of ethanol, added with 42 mg (0.61 mmol) of hydroxylamine hydrochloride and with 0.33 ml (4 mmol) of pyridine. The mixture was heated 1 hr at 80°C, then evaporated, and the residue purified by chromatography with hexane/EtOAc 4:1 to obtain 73 mg of methyl 3-[3'-adamantan-1-yl-4'-methoxy-2-(hydroxyimino-methyl)-biphenyl-4-yl]-acrylate, m.p. 176°C, ¹H-NMR (300 MHz, CDCl₃) δ: 8.15 (1H, s), 8.06 (1H, d, J = 1.83 Hz), 7.75 (1H, d, J = 16.2 Hz), 7.59 (1 H, dd, J = 1.83 Hz, J = 8.24 Hz), 7.41 (1 H, d, J = 7.93 Hz), 7.31 (1 H , br s), 7.12-7.20 (2H, m), 6.96 (1 H, d, J = 8.24 Hz), 6.53 (1 H, d, J = 16.2 Hz), 3.91 (3H, s), 3.84 (3H, s), 2.07-2.16 (9H, m), 1.79 (6H, s).

### Step 2. 3-[3'-Adamantan-1-yl-4'-methoxy-2-(hydroxyiminomethyl)-biphenyl-4-yl]-acrylic acid (16)

Methyl 3-[3'-adamantan-1-yl-4'-methoxy-2-(hydroxyimino-methyl)-biphenyl-4-yl]-acrylate (66 mg, 0.15 mmol) was dissolved in 5.3 ml of 1N NaOH in MeOH,m and the mixture was refluxed 1 hr, evaporated, taken up with water, added with 1M HCl and filtered, to give 51 mg of the product, m.p. 259°C, ¹H-NMR (300 MHz, CDCl₃) δ. 12.4 (1H, s), 11.3 (1H, s), 11.2 (1H, s), 7.97 (1H, s), 7.93 (1H, s), 7.79 (1H, d, J = 8.54 Hz), 7.64 (1H, d, J = 15.9 Hz), 7.40 (1H, d, J = 7.63 Hz), 7.02-7.22 (3H, m), 6.54 (1H, d, J = 15.9 Hz), 3.86 (3H, s), 2.06 (9H, s), 1.72 (6H, s).

### EXAMPLE 17

### 3-[3'-Adamantan-1-yl-4'-methoxy-2-(hydroxymethyl)-biphenyl-4-yl]-acrylic acid (17)

### Step 1. Methyl 3-[3'-adamantan-1-yl-4'-methoxy-2-(hydroxymethyl)-biphenyl-4-yl]-acrylate

A suspension of methyl 3-[3'-adamantan-1-yl-4'-methoxy-2-(formyl)-biphenyl-4-yl]-acrylate (70 mg, 0.16 mmol) in 4 ml of THF/MeOH 1:1 was cooled in an ice bath and treated with 6.4 mg (0.16 mmol) of NaBH₄. After 30 min. at room T, 0.5 M HCl was added (1 ml), and the mixture extracted with AcOEt. Drying, filtering and evaporating gave 70 mg of methyl 3-[3'-adamantan-1-yl-4'-methoxy-2-(hydroxymethyl)-biphenyl-4-yl]-acrylate as a white solid, m.p. 139°C, ¹H-NMR (300 MHz, CDCl₃) δ: 7.77 (1 H, d, J = 16.2 Hz), 7.76 (1 H, d, J = 1.83 Hz), 7.51 (1 H, dd, J = 8.24 and J = 1.83 Hz), 7.33 (1 H, d, J = 8.24 Hz), 7.18-7.23 (2H, m), 6.95 (1 H, d, J = 8.54 Hz), 6.52 (1 H, d, J = 16.2 Hz), 4.69 (2H, s), 3.90 (3H, s), 3.84 (3H, s), 2.07-2.16 (9H, m), 1.75 (6H, s).

### Step 2. 3-[3'-Adamantan-1-yl-4'-methoxy-2-(hydroxymethyl)-biphenyl-4-yl]-acrylic acid (17)

A suspension of methyl 3-[3'-adamantan-1-yl-4'-methoxy-2-(hydroxymethyl)-biphenyl-4-yl]-acrylate (53 mg, 0.123 mmol) in a solution of 26 mg (0.61 mmol) of LiOH.H₂O in 4 ml of THF/water 1:1 was stirred overnight at room T. Evaporation of THF, addition of 2 ml of water, cooling, adding 2M HCl, filtration and drying gave 50 mg of the product, m.p. 226°C, ¹H-NMR (300 MHz, DMSO-*d*₆) δ: 12.4 (1H, br s), 7.80 (1H, s), 7.62 (1H, d, J = 16.2 Hz), 7.61 (1H, dd, J = 8.24 and J = 1.83 Hz), 7.27 (1H, d, J = 8.24 Hz), 7.23 (1H, dd, J = 8.24 and J = 2.14 Hz), 7.17 (1H, d, J = 2.14 Hz), 7.04 (1H, d, J = 8.24 Hz), 6.51 (1H, d, J = 16.2 Hz), 5.21 (1H, br s), 4.42 (2H, s), 3.84 (3H, s), 1.98-2.14 (9H, m), 1.73 (6H, s).

### EXAMPLE 18

### 3-[3'-Adamantan-1-yl-4'-methoxy-2-(3-oxo-but-1-enyl)-biphenyl-4-yl]-acrylic acid (18)

A solution of methyl 3-[3'-adamantan-1-yl-4'-methoxy-2-(formyl)-biphenyl-4-yl]-acrylate (70 mg, 0.16 mmol) in 5 ml of THF/acetone 1:1 was added with LiOH.H₂O (34 mg, 0.82 mmol) and stirred overnight in the dark at room T. Evaporation, taking up with 2N HCl and filtration gave 63 mg of the product, as a yellow solid, m.p. 151-153°C, ¹H-NMR (300 MHz, DMSO) δ: 12.4 (1H, br s), 8.19 (1H, s), 7.79 (1H, dd, J = 7.93 and J = 1.83 Hz), 7.66 (1H, d, J = 16.2 Hz), 7.47 (1H, d, J = 16.2 Hz), 7.46 (1 H, d, J = 7.93 Hz), 7.25 (1 H, dd, J = 8.24 and J = 2.14 Hz), 7.11 (1 H, d, J = 8.24 Hz), 7.07 (1H, d, J = 2.14 Hz), 6.98 (1H, d, J = 16.2 Hz), 6.72 (1H, d, J = 16.2 Hz), 3.87 (3H, s), 2.23 (3H, s), 2.04 (9H, m), 1.71 (6H, s).

### EXAMPLE 19

### Adamantan-1-yl-2-(3-hydroxyimino-but-1-enyl)-4'-methoxy-biphenyl-4-yl]-acrylic acid (19) (RC1238)

A suspension of 3-[3'-adamantan-1-yl-4'-methoxy-2-(3-oxo-but-1-enyl)-biphenyl-4-yl]-acrylic acid (see EXAMPLE. 18) (38 mg, 0.08 mmol) in 0.8 ml of ethanol was added with 17.3 mg (0.25 mmol) of hydroxylamine hydrochloride and 0.135 ml (1.66 mmol) of pyridine, then heated 2 hr. at 80°C. Adding water, cooling in ice, adding 2N HCl to pH 2, and filtration gave 33 mg of the product, m.p. 215°C, ¹H-NMR (300 MHz, DMSO-*d*₆) δ: 12.4 (1 H, br s), 11.2 (1 H, s), 8.06 (1 H, s), 7.62-7.71 (2H, m), 7.37 (1H, d, J = 8.24 Hz), 7.25 (1H, dd, J = 8.24 and J = 2.14 Hz), 7.06-7.13 (2H, m), 6.97 (1H, d, J = 16.5 Hz), 6.80 (1H, d, J = 16.5 Hz), 6.67 (1H, d, J = 16.2 Hz), 3.86 (3H, s), 2.04 (9H, m), 1.83 (3H, s), 1.71 (6H, s).

### EXAMPLE 20

### 3-(3'-Adamantan-1-yl-2-carbamoyl-4'-methoxy-biphenyl-4-yl)-acrylic acid (20)

Step 1. **Methyl 3'-adamantan-1-yl-4-(2-carboxy-vinyl)-4'-methoxy-biphenyl-2-carboxylate.** A suspension of 3-(3'-adamantan-1-yl-2-formyl-4'-methoxy-biphenyl-4-yl)-acrylic acid (70 mg, 0.163 mmol) in 3.6 ml of tert-butanol, was added with 50 mg (0,72 mmol) of 2-methyl-but-2-ene, a solution of 45 mg (0.326 mmol) of NaH₂PO4.H₂O in 0.5 ml of water, and 44 mg (0.49 mmol) of sodium chlorite, left overnight at room T, added with 30 mg of sodium chlorite, then heated 5 h at 60°C. The mixture was treated with satd. aq. NH₄Cl, and filtered, to give the product (71 mg, 97%).
Step 2. **Methyl 3-(3'-adamantan-1-yl-2-carbamoyl-4'-methoxy-biphenyl-4-yl)-acrylate.** A solution of the above acid (70 mg, 0.16 mmol) in dry dichloromethane (3.77 ml) is added with oxalyl chloride (15 µL, 0.17 mmol), and three drops if DMF. After being left at room T for 2 h, the mixture is poured into 10 ml of conc. ice-cooled ammonia. The white precipitate is filtered and purified by chromatography with CH₂Cl2 : CH₃OH 195:5 to give 60 mg (86%) of the product, m.p. 254°C. ¹H-NMR (300 MHz, DMSO-*d*₆): 7.79 (1 H, dd, J = 1.5 Hz, 7.9 Hz), 7.75 - 7.66 (3H, m), 7.46 - 7.36 (2H, m), 7.34 - 7.25 (m, 2H) 7.06 - 6.97 (1H, m), 6.70 (1H, d, J = 16.2 Hz), 3.83 (3H, s), 3.74 (3H, s), 2.14 - 1.97 (m, 9H), 1.82 - 1.68 (m, 6H).
Step 3. **3-(3'-Adamantan-1-yl-2-carbamoyl-4'-methoxy-biphenyl-4-yl)-acrylic acid.** A suspension of 45 mg (0.1 mmol) of the above ester was suspended in 3.2 ml of 50% aq. THF, added with 21 mg (0.5 mmol) of LiOH.H₂O, and left 72 hr at room T in the dark. Evaporation of THF, ice-cooling, addition of 1N HCl, and filtration gave 32 mg (73%) of the product, m.p. 265°C (dec). ¹H-NMR (300 MHz, DMSO-*d*₆): 12.4 (1 H, brs), 7.82 - 7.52 (4H, m), 7.47 - 7.33 (2H, m), 7.33 - 7.18 (2H, m), 7.00 - 6.93 (1H, m), 6.57 (1H, d, J = 16.8 Hz), 3.83 (3H, s), 2.13 - 1.93 (9H, m), 1.81 - 1.60 (6H, m).

### EXAMPLE 21

### 3-[3'-Adamantan-1-yl-4'-(3-hydroxycarbamoyl-propoxy)-biphenyl-4-yl]-acrylic acid (21).

Step 1. **3-[3'-Adamantan-1-yl-4'-(3-ethoxycarbonyl-propoxy)-biphenyl-4-yl]-acrylic** acid tert-**butyl ester.** A mixture of 3-(3'-adamantan-1-yl-4'-hydroxy-biphenyl-4-yl)-acrylic acid tert-butyl ester (50 mg, 0.115 mmol), ethyl bromobutanoate (36 mg, 0.17 mmol), K₂CO₃ (48 mg, 0.345 mmol) in 1.4 ml of DMF was heated at 60-80°C for 2 h, added with 12 mg of ethyl bromobutanoate and heated again for 6 h. Evaporation, taking up with EtOAc, washing with water, dryng, evaporation and chromatography with hexane/EtOAc 9:1 gave 67 mg of the product, m.p. 148°C. ¹H-NMR (300 MHz, CHCl₃-*d*): 7.79 - 7.46 (6H, m), 7.41 (1H, dd, J = 1.8, 8.2 Hz), 6.94 (1H, d, J = 8.2 Hz), 6.40 (1 H, d, J = 15.9 Hz), 4.28 - 4.01 (4H, m), 2.64 (2H, t, J = 7.6 Hz), 2.30 - 2.03 (11 H, m), 1.87 - 1.70 (6H, m), 1.56 (s, 9H), 1.30 (3H, t, J = 7.3).
Step 2. **3-[3'-Adamantan-1-yl-4'-(3-carboxy-propoxy)-biphenyl-4-yl]-acrylic acid tert-butyl ester.** The above ester (59 mg, 0.11 mmol), was dissolved in 3.4 ml of aq. 50% THF, added with 14 mg (0.32 mmol) of LiOH. H₂O, and the mixture was left overnight in the dark. Evaporation of the solvent, taking up with EtOAc, washing with 1 N H₂SO₄ (10 ml), drying and evaporation gave 43 mg (87%) of the product as a white solid, m.p. >280°C. ¹H-NMR (300 MHz, CDCl₃): 7.37 /1 H, d, J = 16.2 Hz), 7.33 - 7.26 84H, m), 7.24 (1H, d, J = 2.1 Hz), 7.17 81 H, dd, 2.1, 8.2 Hz), 6.69 (1 H, d, J = 8.2 Hz), 6.15 (1 H, d J =16.2 Hz), 3.87 (2H, t, J = 6.1 Hz), 2.47 (2H, t, J = 7.3 Hz), 2.06 - 1.95 (2H, m), 1.94-1.83 (9H, m), 1.59 - 1.49 (6H, m) 1.31 (9H, s).
Step 3. **3-{3'-Adamantan-1-yl-4'-[3-(tetrahydro-pyran-2-yloxycarbamoyl)-propoxy]-biphenyl-4-yl}-acrylic acid tert-butyl ester.** A mixture of the ebove ester (48 mg, 0.093 mmol), HOBt (26 mg, 0,19 mmol), and WSC (37 mg, 0.19 mmol)in 1 ml of dry DMF is left 5 h at room T, then is added with 23 mg (0.19 mmol) of O-(tetrahydro-pyran-2-yl)-hydroxylamine, and left overnight. Evaporation and chromatography with hexane/EtOAc 6:4 gave 153 mg of the product, that was used for the following step without further purification. ¹H-NMR (300 MHz, CDCl₃): 7.97 (1H, brs), 7.38 (1 H, d, J = 16.2 Hz), 7.34 - 7.28 (4H, m), 7.24 (1 H, d, J = 1.8 Hz), 7.17 (1 H, dd, J = 1.8, 8.2 Hz), 6.69 (1H, d, J = 8.24 Hz), 6.14 (1 H, d, J = 16.2 Hz), 4.78 - 4.66 (1 H, m), 3.86 (2H, t, J = 5.8 Hz), 3.73 - 3.60 (1 H, m), 3.43 - 3.30 (1 H, m), 2.27 - 2.12 (2H, m), 2.09 - 1.97 (2H, m), 1.96 - 1.82 (9H, m), 1.67- 1.48 (10H, m), 1.45 - 1.27 (11H, m)
Step 4. **3-[3'-Adamantan-1-yl-4'-(3-hydroxycarbamoyl-propoxy)-biphenyl-4-yl]-acrylic acid.** Into an ice-cooled solution of the above ester (41 mg, 0.07 mmol) in 1.4 ml of CH₂Cl₂ was dropped TFA (0.7 ml). The mixture was left 2 h at 0°C, then evaporated, to give 42 mg of a yellow solid, that was chromatographed with CH₂Cl₂ : CH₃OH:H₂O 18:2:0.2 to give 14 mg (44%) of the product as a white solid m.p. 242°C (dec). ¹H-NMR (300 MHz, DMSO-*d*₆): 10.47 (1H, s), 8.75 (1H, brs), 7.76 - 7.54 (5H, m), 7.51 (1H, dd, J = 1.8, 8.5 Hz), 7.43 (1H, d, J = 1.8 Hz), 7.03 (1H, d, J = 8.5 Hz), 6.53 (1 H, d, J = 16.2 Hz), 4.03 (2H, t, J = 7.0 Hz), 2.68-2.55 (2H, m), 2.29-2.17 (2H, m), 2.16-1.95 (9H, m), 1.82 - 1.66 (6H, m).

### EXAMPLE 22

### 3-[3'-Adamantan-1-yl-4'-(4-hydroxycarbamoyl-butoxy)-biphenyl-4-yl]-acrylic acid.

Step 1. **3-[3'-Adamantan-1-yl-4'-(4-ethoxycarbonyl-butoxy)-biphenyl-4-yl]-acrylic acid tert-butyl ester.** A solution of 3-(3'-adamantan-1-yl-4'-hydroxy-biphenyl-4-yl)-acrylic acid tert-butyl ester (200 mg, 0.46 mmol) and of ethyl 5-bromovalerate (194 mg, 0.92 mmol) in 8 ml of DMF was added with K₂CO₃ (257 mg, 1.86 mmol) and heated 3.5 h at 80°C in the dark. Evaporation and chromatography with hexane/EtOAc 9:1 gave 197 mg (76%) of the product, m.p. 127°C. ¹H-NMR (300 MHz, CDCl₃) δ: 7.62 (1 H, d, J = 15.8), 7.58 - 7.53 (4H, m), 7.48 (1 H, d, J = 2.2 Hz), 7.41 (1 H, dd, J = 2.2, 8.4 Hz), 6.93 (1H, d, J = 8.4), 6.38 (1H, d, J = 15.8 Hz), 4.16 (2H, d, j = 7.2), 4.08 - 4.00 (2H, m), 2.47 - 2.38 (2H, m), 2.24 - 2.06 (9H, m), 1.99 - 1.88 (4H, m), 1.84 - 1.74 (6H, m), 1.55 (9H, s), 1.27 (3H, t, J = 7.2 Hz)
Step 2. **3-[3'-Adamantan-1-yl-4'-(4-carboxy-butoxy)-biphenyl-4-yl]-acrylic acid tert-butyl ester.** A solution of the above ester (170 mg, 0.3 mmol) in 9,6 ml of aq. 50% THF was added with 38 mg (0.31 mmol) of LiOH.H₂O and left 3 h. Evaporation, taking up with EtOAc, washing with 20ml of 1N KHSO₄, extraction with EtOAc, drying the extract with Na₂SO₄, filtration and evaporation gave 136 mg (84%) of the product, m.p. 169°C. ¹H-NMR (300 MHz, DMSO-*d*₆) δ: 12.06 (1H, bs), 7.76 - 7.61 84H, m) 7.57 (1H, d, J = 15.9 Hz), 7.51 (1H, d, J = 2.3, 8.6 Hz), 7.43 (1H, d, J = 2.3 Hz), 7.05 (1H, d, J = 8.6 Hz), 6.53 (1H, d, J = 15.9 Hz), 4.03 (2H, t, J = 5.9 Hz), 2.32 (2H, t, J = 6.6 Hz), 2.17 - 1.99 (9H, m), 1.88 - 1.68 (10H, m), 1.49 (9H, s).
Step 3. **3-{3'-Adamantan-1-yl-4'-[4-(tetrahydro-pyran-2-yloxycarbamoyl)-butoxy]-biphenyl-4-yl}-acrylic acid tert-butyl ester.** A mixture of the above ester (130 mg, 0.245 mmol), HOBt (68 mg, 0,49 mmol), and WSC (37 mg, 0.19 mmol) in 1 ml of dry DMF is left 3 h at room T, added again with 1 ml of DMF and 24 mg of WSC, left overnight, then added with 85 mg (1.22 mmol) of O-(tetrahydro-pyran-2-yl)-hydroxylamine HCl, and 123 (1.22 mmol) of triethylamine and left 3 h under nitrogen atmosphere and in the dark. Evaporation of the DMF, taking up with 3 ml of water, filtration and chromatography with CH₂Cl₂ : CH₃OH 195:5, and a second chromatography with CH₂Cl₂ : CH₃OH 190:10 gave 60 mg (45%) of the product as a white solid. ¹H-NMR (300 MHz, DMSO-*d*₆) δ: 10.39 (1 H, s), 8.71 (1 H, s), 7.77 - 7.61 (4H, m), 7.58 (1 H, d, J = 16.0 Hz), 7.51 (1 H, dd, J = 2.2, 8.7 Hz), 7.43 (1 H, d, J = 2.2 Hz), 7.05 (1 H, d, J = 8.7 Hz), 6.53 (1 H, d, J = 16.0 Hz), 4.07 - 3.97 (2H, m), 2.18 - 1.98 ()H, m), 1.86 - 1.66 (10H, m) 1.49 (9H, s).
Step 4. **3-[3'-Adamantan-1-yl-4'-(4-hydroxycarbamoyl-butoxy)-biphenyl-4-yl]-acrylic acid.** Into an ice-cooled solution of the above ester (52 mg, 0.095 mmol) in 1.9 ml of CH₂Cl₂ was dropped TFA (0.95 ml). The mixture was left 3 h at 0°C, under nitrogen and in the dark, then evaporated, to give 42 mg, 89%) of the product as a white solid m.p. 224°C (dec). ¹H-NMR (300 MHz, DMSO-*d*₆) δ: 13.38 (1 h, bs), 7.78 - 7.55 (5H, m), 7.51 (1H, dd, J = 2.2, 8.3 Hz), 7.43 (1H, d, J = 2.2 Hz), 7.05 (1 H, d, J = 8.3 Hz), 6.54 (1 H, d, J = 15.7 Hz), 4.07 - 3.97 (2H, m), 2.22 - 1.98 (11 H, m), 1.91 - 1.62 (10H, m).

### EXAMPLE 23

### 3-[3'-Adamantan-1-yl-4'-(7-hydroxycarbamoyl-heptyloxy)-biphenyl-4-yl]-acrylic acid (23)

Step 1. **Tert-butyl 3-{3'-adamantan-1-yl-4'-[7-(tetrahydro-pyran-2-yloxycarbamoyl)-heptyloxy]-biphenyl-4-yl}-acrylate.** A mixture of tert-butyl 3-(3'-adamantan-1-yl-4'-hydroxybiphenyl-4-yl)-acrylate (150 mg, 0.35 mmol), 8-bromo-octanoic acid (tetrahydro-pyran-2-yloxy)-amide (146 mg, 0.45 mmol), and K₂CO₃ (137 mg, 0.99 mmol) in 6 ml of dry acetonitrile was refluxed 12 hrs, then added with 1 ml of dry DMF and 80 mg of K₂CO₃ and again refluxed 5 hrs. Evaporation, filtration and drying gave a crude product, that was purified by chromatography with hexane/AcOEt 1:1 to give 100 mg of a white solid, m.p. 154-156°C, ¹H-NMR (300 MHz, CDCl₃) δ: 8.16 (1H, br s), 7.45-7.67 (6H, m), 7.42, 1H, dd, J = 8.24 and J = 2.14 Hz), 6.34 (1H, d, J = 8.24 Hz), 6.33 (1H, m, J = 16.2 Hz), 4.96 (1H, br s), 3.89-4.07 (3H, m), 3.60-3.70 (1H, m), 2.07-2.24 (11H, m), 1.75-1.96 (12H, m), 1.51-1.74 (15H, m), 1.36-1.48 (4H, m).
Step 2. **3-[3'-Adamantan-1-yl-4'-(7-hydroxycarbamoyl-heptyloxy)-biphenyl-4-yl]-acrylic acid (23)**
   A solution of tert-butyl 3-{3'-adamantan-1-yl-4'-[7-(tetrahydro-pyran-2-yloxycarbamoyl)-heptyloxy]-biphenyl-4-yl}-acrylate (88 mg, 0.13 mmol) in 2.6 ml of CH₂Cl₂ was cooled in an ice bath, treated dropwise with 1.3 ml of TFA and stirred at room T for 30 min. Evaporation, and taking up with ether gave a white solid, that was washed with hexane to give 70 mg of the product, m.p. 177°C (dec), ¹H-NMR (300 MHz, DMSO-*d*₆) δ: 12.3 (1 H, br s), 10.3 (1 H, s), 8.66 (1 H, brs), 7.56-7.77 (5H, m), 7.51, 1H, d, J = 8.24 Hz), 7.43 (1H, s), 7.04 (1H, d, J = 8.24 Hz), 6.53 (1H, m, J = 16.2 Hz), 4.02 (2H, t, J = 5.19 Hz), 2.01-2.18 (9H, m), 1.94 (2H, t, J = 7.93 Hz), 1.67-1.87 (8H, m), 1.43- 1.60 (4h, m), 1.19-1.42 (4H, m).

### EXAMPLE 24

### 2-(2-{4-[3-Adamantan-1-yl-4'-(2-carboxy-vinyl)-biphenyl-4-yloxy]-butyrylamino}-ethyldisulfanyl)-ethyl-ammonium; trifluoroacetate (24) (RC 1363)

Step 1. **3-[3'-Adamantan-1-yl-4'-(3-{2-[2-tert-butoxycarbonylamino-ethyldisulfanyl]-ethylcarbamoyl}-propoxy)-biphenyl-4-yl)-acrylic acid tert-butyl ester.** A mixture of 3-[3'-Adamantan-1-yl-4'-(3-carboxy-propoxy)-biphenyl-4-yl]-acrylic acid tert-butyl ester (80 mg, 0.155 mmol), HOBt (43 mg, 0.31 mmol), and WSC (61 mg, 0.31 mmol) in 0.84 ml of DMF was stirred for 5 h (a precipitate formed), then added with [2-(2-amino-ethyldisulfanyl)-ethyl]-carbamic acid tert-butyl ester (Biochem. Biophys, Res. Comm. 331, 1, 351-356) (the precipitate dissolves) and left overnight. Evaporation and chromatography with hexane/EtOAc 4:6 gave 99 mg (85%) of the product as a white solid, m.p. 79°C. ¹H-NMR (300 MHz, CDCl₃): 7.62 (1 H, d, J = 16.0), 7.61 - 7.51 (4H, m), 7.48 (1 H, d, J = 2.3), 7.40 (1 H, dd, J = 2.3, 8.3), 6.93 (1 H, d, J = 8.3), 6.38 (1 H, d, J = 16.0), 6.42 (1 H, bs), 4.94 (1 H, bs), 4.08 (2H, t, J = 6.1), 3.65 - 3.55 (2H, m), 3.49 - 3.38 (2H, m), 2.85 (2H, t, J = 6.2), 2.76 (2H, t, J = 6.9), 2.52 (2H, t, J = 7.2), 2.32 - 2.20 (2H, m), 2.18 - 2.06 (9H, m), 1.85 - 1.74 (6H, m), 1.61- 1.52 (9H, s), 1.45 (9H, s).
Step 2. **2-(2-{4-[3-Adamantan-1-yl-4'-(2-carboxy-vinyl)-biphenyl-4-yloxy]-butyrylamino}-ethyldisulfanyl)-ethyl-ammonium; trifluoroacetate.**
A solution of the above ester (81 mg, 0.11 mmol) in 8.2 ml of dichloromethane was cooled with ice, then added with dropwise with 2 ml of TFA, and left at 0°C under nitrogen, for 4 h. Evaporation gave 76 mg (100%) of the product, as a yellow solid, m.p 140°C. ¹H-NMR (300 MHz, CH₃OH-*d₄*): 7.77 (6H, m), 7.51 - 7.40 (2H, m), 7.01 (1H, d J = 8.5), 6.49 (1H, d, J 0 15.9), 4.09 (2H, t, J = 6.2), 3.54 (2H, t, J = 6.7), 3.28 (2H, t, J = 6.7), 2.97 (2H, t, J = 6.7), 2.87 (2H, t, J = 6.7), 2.51 (2H, t, J = 7.0), 2.26 - 2.14 (9H, m), 2.13 - 2.03 (2H, m), 1.89 - 1.77 (6H, m).

### EXAMPLE 25

### 3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propoxy]-biphenyl-4-yl}-acrylic acid. (RC 1338)

Step 1. **(2-{2-[3-(3-Bromo-4-hydroxy-phenyl)-2-hydroxyimino-propionylamino]-ethyldisulfanyl}-ethyl)-carbamic acid tert-butyl ester.** A solution of 3-(3-bromo-4-hydroxy-phenyl)-2-hydroxyimino-propionic acid (1.54 g, 5.62 mmol) in 34 ml of DMF
   was added with DCC (1.74 g, 8.43 mmol) and N.hydroxysuccinimide (0.99 g, 8.43 mmol) and left at room T for 3 h, then added with [2-(2-amino-ethyldisulfanyl)-ethyl]-carbamic acid tert-butyl ester, and left overnight at room T. Evaporation, cooling, addition of dichloromethane, filtration, evaporation and chromatography with CH₂Cl₂ : CH₃OH 95.5 gave 984 mg of the product, which was used without further purification. ¹H-NMR (300 MHz, CDCl₃): 7.47 (1 H, d, J = 1.8), 7.22 (1 H, dd, J 0 1.8, 8.5), 6.89 (1 H, d J = 8.5 Hz), 5.10 (1 H, bs), 5.91 (1 H, bs), 3.88 (2H, s), 3.71 - 3.57 (2H, m), 3.50 - 3.35 (2H, m), 2.93 - 2.68 (4H, m), 1.46 (9H, s).
Step 2. **2-{2-[3-(3-Bromo-4-hydroxy-phenyl)-2-hydroxyimino-propionylamino]-ethyldisulfanyl}-ethyl-ammonium; trifluoro-acetate.** A solution of the above compound (0.97 g, 1.9 mmol) in 61 ml of dichloromethane, under nitrogen, was added with 3.7 ml of TFA, and left 2 h at room T. Evaporation and chromatography with CH₂Cl₂ : CH₃OH 90:10, then with CH₂Cl₂ : CH₃OH 85:15, gave 938 mg of the product. ¹H-NMR (300 MHz, DMSO-*d*₆): 8.12 (1H, t, J = 5.2 Hz), 7.28 (1H, d, J = 1.8 Hz), 7.01 (1H, dd, J = 1.8, 8.2 Hz), 6.83 (1H, d, J = 8.2 Hz), 3.69 (2H, s), 3.49 - 3.39 (2H, m), 2.97 (2H, t, J = 6.4 Hz), 2.87 - 2.78 (4H, m).
Step 3. **3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propoxy]-biphenyl-4-yl}-acrylic acid tert-butyl ester.** A mixture of 3-[3'-adamantan-1-yl-4'-(4-carboxy-propoxy)-biphenyl-4-yl]-acrylic acid tert-butyl ester (65 mg, 0.126 mmol), HOBt (35 mg, 0.25 mmol), and WSC (49 mg, 0.25 mmol) in 1 ml of DMF was stirred for 3 h (a white precipitate formed), added with a solution of 98 mg (0.19 mmol) of 2-{2-[3-(3-bromo-4-hydroxy-phenyl)-2-hydroxyimino-propionylamino]-ethyldisulfanyl}-ethyl-ammonium; trifluoroacetate in 0.9 ml of dry DMF, and left at room T under nitrogen, for 72 h. Evaporation and chromatography with CH₂Cl₂ : CH₃OH 20:0.4 gave 71 mg of the product, as a mixture of syn and anti-oxime. ¹H-NMR (300 MHz, CDCl₃): 9.34 (1H, brs), 7.61 (1H, d, J = 16.1 Hz), 7.56-7.51 (4H, m), 7.49 - 7.43 (2H, m), 7.37 (1 H, dd, J = 1.9, 8.2 Hz), 7.24 - 7.16 (2h, m), 6.90 - 6,84 (2h, m), 6.38 (1H, d, J = 16.1), 6.2 (1H, t, J = 5.9 Hz), 5.59 (1H, bs), 4.06 (2H, t, J = 5.9), 3.86 (2H, s), 3.66 - 3.53 (4H, m), 2.87 - 2.76 (4H, m), 2.54 (2H, t, J = 7.3), 2.32 - 2.20 (2H, m), 2.19 - 2.04 (9H, m), 1.86 - 1.70 (6H, m), 1.55 (9H, s).
Step 4. **3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propoxy]-biphenyl-4-yl}-acrylic acid.** A solution of the above ester (61 mg, 0,07 mmol) in 9 ml of dichloromethane was cooled in ice, then added dropwise with 1.5 ml of TFA, and left at room T for 4 h. Evaporation, and crystallization from dichloromethane/ether gave 46 mg of the product, m.p. 169°C (dec). ¹H-NMR (300 MHz, DMSO-*d₆):* 12.32 (1H, bs), 11.84 (1H, s), 10.02 (1H, s), 8.15 - 7.99 (1H, m), 7.77 - 7.55 (5H, m), 7.49 81 H, dd, J = 1.7, 8.3), 7.42 (1 H, d, J = 1.7), 7.26 (1 H, s), 7.07 - 6.94 (2H, m), 6.81 (1 H, d, J = 8.3), 6.51 (1 H, d, J = 16.1), 4.07 - 3.96 (2H, m), 3.66 (2H, s), 3.52 - 3.37 (4H, m), 2.85 - 2.72 (4H, m), 2.33 (2H, t, J = 7.3), 2.18 - 1.99 (11 H, m), 1.78 - 1.66 (6H, m).

### EXAMPLE 26

### 3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propionyloxy]-biphenyl-4-yl}-acrylic acid (26) (AB 514)

Step 1. **3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propionyloxy]-biphenyl-4-yl}-acrylic acid tert-butyl ester. (AB513)** A solution of succinic acid mono-[3-adamantan-1-yl-4'-(2-tertbutoxycarbonyl-vinyl)-biphenyl-4-yl] ester (70 mg) in 2 ml of DMF was added with 35 mg of WSC and 22 mg of HOBt, and left overnight at room T under a nitrogen atmosphere. Evaporation and chromatography with CH₂Cl₂ : CH₃OH 20:1 gave 90 mg of the title product, ¹H NMR (300 MHz, CDCl₃): 7.61 (1H, d, J = 15.9 Hz), 7.57 - 7.52 (5H, m), 7.44 (1H, d, J = 2.5 Hz), 7.41 - 7.35 (1H, m), 7.18 (1H, dd, J = 2.5, 8.7 Hz), 7.04 (1H, d, J = 8.3 Hz), 6.86 (1H, d, J = 8.3 Hz), 6.39 (1H, d, J = 15.9 Hz), 3.85 (2H, s), 3.63 - 3.48 (4H, m), 3.05 - 2.96 (2H, m), 2.84 - 2.74 (4H, m), 2.72 - 2.60 (2H, m), 2.13 - 1.99 (9H, m), 1.84 - 1.69 (6H, m), 1.55 (9H, s).
Step 2. **3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propionyloxy]-biphenyl-4-yl}-acrylic acid. (AB514)** A solution of 3-{3'-adamantan-1-yl-4'-[3-(2-]2-[3-(3-bromo-4-hydroxy-phenyl)-2-hydroxyimino-propionylamino]-ethyldisulfanyl}-ethylcarbamoyl)-propionyloxy]-biphenyl-4-yl}-acrylic acid tert-butyl ester (75 mg) in 10 ml of dry dichloromethane was cooled at 0°C, added dropwise with 1.5 ml of TFA and stirred 3 h. Concentration and chromatography with CH₂Cl₂ : CH₃OH 20:1, then with CH₂Cl₂ : CH₃OH 10:1 gave a crude product, that was dissolved in a mixture of dichloromethane and methanol, then added with ethyl ether, to give a precipitate, that was filtered, to give 50 mg of the title product, m.p, 198-199°C (dec),

¹H-NMR (300 MHz, DMSO-*d*₆): 11.92 (1 H, bs), 9.63 (1 H, bs), 8.19 (1 H, t, J = 5.8 Hz), 8.10 (1 H, t, J = 5.8 Hz), 7.17 - 7-47 (5H, m), 7.43 - 7.22 (3H, m), 7.08 (1H, d, J = 8.5 Hz), 7.00 (1H, d, J = 8.8 Hz), 6.90 - 6.81 (1H, m), 6.54 - 6.41 (1H, m), 3.68 (2H, s), 2.91 - 2.70 (6H, m), 2.58 - 2.52 (2H, m), 2.19 - -1.92 (9H, m), 1.82 - 1.67 86H, m).

### EXAMPLE 27

### 3-[3'-Adamantan-1-yl-4'-hydroxy-2-(7-hydroxycarbamoyl-heptylcarbamoyl)-biphenyl-4-yl]-acrylic acid (27) (RC 1207).

Step 1. **3-[3'-Adamantan-1-yl-4'-(tert-butyl-dimethyl-silanyloxy)-2-(7-carboxy-heptylcarbamoyl)-biphenyl-4-yl]-acrylic acid methyl ester.** A solution of **3'**-adamantan-1-yl-4'-(tert-butyl-dimethyl-silanyloxy)-4-(2-methoxycarbonyl-vinyl)-biphenyl-2-carboxylic acid (see Example 5) (100 mg, 0.183 mmol) in 3.7 ml of THF was added with DCC (64 mg, 0.31 mmol) and N.hydroxysuccinimide (115 mg, 0.33 mmol) and left at room T overnight, then added with 8-aminooctanoic acid (73 mg, 0.46 mmol) and triethylamine (92 mg, 0.91 mmol) and heated 8 h at 60-70°C. Evaporation and chromatography with CH₂Cl₂ : CH₃OH 197:3 gave 100 mg of the title compound. m.p. 149°C. ¹H-NMR (300 MHz, CH₃OH-*d₄*): 7.95 (1 H, t, J = 6.4 Hz), 7.75 - 7.58 (3H, m), 7.41 (1 H, d, J = 7.9 Hz), 7.30 - 7.23 (1 H, m), 7.18 - 7.11 (1 H, m), 6.86 (1 H, d, J = 8.2 Hz), 6.56 (1H, d, J = 15.8 Hz), 3.88 (3H, s), 3.12 (2H, m), 2.22 (2H, m), 2.17 - 1.98 (9H, m), 1.86 - 1.71 (6H, m), 1.65 - 1.47 (2H, m), 1.41 - 1.17 (6H, m), 1.05 (9H, s), 0.96 - 0.75 (2H, m), 0.35 (6H, s).
Step 2. **3-[3'-Adamantan-1-yl-4'-(tert-butyl-dimethyl-silanyloxy)-2-(7-hydroxycarbamoyl-heptylcarbamoyl)-biphenyl-4-yl]-acrylic acid methyl ester**
   A suspension of 3-[3'-Adamantan-1-yl-4'-(tert-butyl-dimethyl-silanyloxy)-2-(7-carboxyheptylcarbamoyl)-biphenyl-4-yl]-acrylic acid methyl ester (98 mg, 0.14 mmol) in 2.9 ml of dry THF was added with DCC (50 mg, 0.24 mmol) and N-hydroxysuccinimide (30 mg, 0.26 mmol), left overnight at room T, then added with hydroxylamine.HCl (25 mg, 0.36 mmol) and triethylamine (72 mg, 0.71 mmol), and left again overnight at room T.

The mixture was added with 1 N HCl and extracted with EtOAc. Evaporation and chromatography with CH₂Cl₂ : CH₃OH 195 : 5 to 190 : 10 gave 34 mg of the title compound. m.p. 93°C. ¹H-NMR (300 MHz, CH₃OH-*d₄*): 7.82 - 7.57 (3H, m), 7.43 (1 H, dd, J = 7.9, 1.8 Hz), 7.27 (1 H, d, J = 1.8 Hz), 7.17 (1H, dd, J = 2.4, 8.8 hz), 6.87 (1H, J = 1.8, 7.9), 6.57 (1H, d, J = 15.9 Hz), 3. 78 (3H, s), 3.13 (2H, t, J = 7.0 Hz), 2.20 - 1.98 (11 H, m), 1.86 - 1.75 (6H, m), 1.63 - 1.48 (2H, m), 1.38 - 1.48 (2H, m), 1.38 - 1.11 (6H, m), 1.07 (9H, s), 0.94 - 0.77 (2H, m), 0.37 (6H, s).

Step 3. **3-[3'-Adamantan-1-yl-4'-hydroxy-2-(7-hydroxycarbamoyl-heptylcarbamoyl)-biphenyl-4-yl]-acrylic acid.** A solution of 3-[3'-adamantan-1-yl-4'-(tert-butyl-dimethyl-silanyloxy)-2-(7-hydroxycarbamoyl-heptylcarbamoyl)-biphenyl-4-yl]-acrylic acid methyl ester (32 mg, 0.05 mmol) in 1.8 ml of 1 N NaOH in methanol was refluxed for 5 h. Evaporation, cooling with ice, adding 2 ml of 2N HCl, and filtration gave 16 mg of the title product as a white solid, m.p. 144°C. ¹H-NMR (300 MHz, CH₃OH-*d₄*): 7.88 (1 H, t, J = 6.1 hz), 7.74 - 7.56 (3H, m), 7.46 - 7.32 (2H, m), 7.24 - 7.14 (1 H, m), 7.13 - 7.03 (1 H, m), 6.82 - 6.68 (1 H, m), 6.51 (1 H, d, J = 16.2), 3.18 - 3.02 (2H, m), 2.10 - 1.96 (11 H, m), 1.88 - 1.70 (6H, m), 1.65 - 1.48 (2H, m), 1.42 - 1.14 (6H, m), 1.4 - 0.97 (2H, m).

### EXAMPLE 28

### 3-[3'-adamantan-1-yl-4'-(4-carboxy-butoxy)-biphenyl-4-yl]-acrylic acid (28)

TFA (0.77 mL) was dropped into a solution of 3-[3'-adamantan-1-yl-4'-(4-carboxy-butoxy)-biphenyl-4-yl]-acrylic acid *tert*-butyl ester (40 mg, 0.077 mmol) (in CH₂Cl₂ (2.44 mL) at 0 °C (see example 22, step 2). The resulting yellow solution was stirred 3h at 0 °C. Evaporation followed by crystallization from CH₂Cl₂ gave the title compound as a white solid, m.p. 274°C; Yield 95%; R*_{f}* 0.10 (MeOH, RP). ¹H-NMR (DMSO-*d*₆) δ: 12.05 (1H, brs); 7.75-7.60 (4H, m); 7.59 (1H d, J= 15.8 Hz); 7.49 (1 H, dd, J= 8.6 Hz, J= 2.1 Hz); 7.41 (1 H, d, J= 2.1 Hz); 7.03 (1 H, d, J= 8.6 Hz); 6.52 (1 H, d, J= 15.8 Hz); 4.01 (2H, t, J= 5.5 Hz), 2.30 (2H, t, J= 6.9 Hz); 2.15-1.99 (9H, m); 1.87-1.66 (10H, m).

### EXAMPLE 29

### 3-(3'-Adamantan-1-yl-4'-hydroxycarbamoylmethoxybiphenyl-4-yl)-acrylic acid (MIR002) (29) Step 1: 3-(3'-Adamantan-1-yl-4'-ethoxycarbonylmethoxy-biphenyl-4-yl)-acrylic acid tert-butyl ester

A solution of 3-(3'-Adamantan-1-yl-4'-hydroxybiphenyl-4-yl)acrylic acid tert-butyl ester (250 mg, 0.581 mmol) and K₂CO₃ (321 mg, 2.32 mmol) in anhydrous DMF (10 mL), was added with ethyl bromoacetate (200 mg, 1.16 mmol). The solution was stirred at 80°C under nitrogen for 2h. Filtration of potassium carbonate, followed by evaporation and purification by flash chromatography with ETP/AcOEt 90 : 10 gave 224 mg of the title compound. Yield 75%. R*_{f}* 0.31 (ETP/AcOEt 9:1). ¹H-NMR (300 MHz, CDCl₃) δ: 7.61 (1 H, d, J = 16.1 Hz); 7.57-7.54 (4H, m); 7.50 (1 H, d, J = 2.4 Hz); 7.39 (1 H, dd, J = 2.4, 8.4 Hz); 6.80 (1 H, d, J = 8.4 Hz); 6.38 (1 H, d, J= 16.1 Hz); 4.67 (2H, s); 4.31 (2H, q, J = 7.1 Hz); 2.26-2.05 (9H, m); 1.80 (6H, s); 1.54(9H, m); 1.32 (3H, t, J = 7.1 Hz).

### Step 2: 3-(3'-Adamantan-1-yl-4'-carboxymethoxy-biphenyl-4-yl)acrylic acid tert-butyl ester

A suspension of 3-(3'-adamantan-1-yl-4'-ethoxycarbonylmethoxy-biphenyl-4-yl)-acrylic acid tert-butyl ester (191 mg, 0.370 mmol) in THF/H₂O 1:1 (11.4 mL) was added with LiOH.H₂O (77.6 mg, 1.85 mmol). The mixture was stirred at room temperature overnight. Evaporation, addition of ethyl acetate, washing with KHSO₄ 1N, drying and evaporation gave 181 mg of the title compound. Yield: 100%. R*_{f}* 0.23 (CH₂Cl₂ : MeOH 19:1). ¹H-NMR (300 MHz, DMSO-*d*₆) δ: 7.78-7.37 (7H, m); 6.93 (1H, d, J = 8.6 Hz); 6.50 (1H, d, J= 16.1 Hz); 4.71 (2H, s); 2.18-1.95 (9H, m); 1.73 (6H, s); 1.48 (9H, m).

### Step 3: 3-(3'-Adamantan-1-yl-4'-hydroxycarbamoylmethoxy-biphenyl-4-yl)acrylic acid tert-butyl ester

A solution of 3-(3'-adamantan-1-yl-4'-carboxymethoxybiphenyl-4-yl)acrylic acid tert-butyl ester (153 mg, 0.313 mmol), WSC (92 mg, 0.470 mmol), N-hydroxysuccinimide (55.2 mg, 0.470 mmol) in anhydrous DMF (3.30 mol) was stirred at room temperature overnight, then hydroxylamine hydrochloride (109 mg, 1.57 mmol) and TEA (159 mg, 1.57 mmol) were added. After stirring at room temperature for 12 h, the solvent was evaporated and water (5 mL) was added. Filtration and purification by chromatography (CH₂Cl₂/MeOH 19 :1) gave 84 mg of the title compound. Yield 53%.

R*_{f}* 0.54 (CH₂Cl₂ : MeOH 19:1) ¹H-NMR (300 MHz, DMSO-*d*₆) δ: 10.73 (1 H,s); 9.02 (1 H, s); 7.82-7.37 (7H, m); 6.98 (1H, d, J = 8.8 Hz); 6.51 (1H, d, J= 16.1 Hz); 4.47 (2H, s); 2.18-1.95 (9H, m); 1.73 (6H, s); 1.48 (9H, m)..

### Step 4: 3-(3'-Adamantan-1-yl-4'-hydroxycarbamoylmethoxybiphenyl-4-yl)-acrylic acid

A solution of 3-(3'-Adamantan-1-yl-4'-hydroxycarbamoylmethoxy-biphenyl-4-yl)acrylic acid tert-butyl ester (61 mg, 0.121 mmol) in CH₂Cl₂ dry (3.81 mL) was added with TFA (1.21 mL) and stirred at 0°C for 2h under nitrogen. The solvent was evaporated, toluene (2 x 3 mL) was added and the solvent was removed by azeotropic distiilation. Crystallization from CH₂Cl₂ gave 46 mg of the title compound as a white solid, m.p. 265°C; Yield 85%; R*_{f}* 0.10 (CH₂Cl₂ : MeOH 18:2). ¹H-NMR (300 MHz, DMSO-*d*₆) δ: 12.34 (1H, s); 10.77 (1H,s); 9.05 (1H, s); 7.81-7.31 (7H, m); 7.06-6.90 (1H, m); 6.52 (1 H, d, J= 16.5 Hz); 4.45 (2H, s); 2.22-1.99 (9H, m); 1.72 (6H, s).

### EXAMPLES 30-33

### Evaluation of biological activity

### Cell lines

Human NCI-H460 NSCLC, H460-R9A (Adarotene-resistant NCI-H460) NSCLC, A2780 and A2780-Dx (multidrug resistant) ovarian carcinoma, MDA-MB436 breast carcinoma, MM288, MM317, MM404, MM473, and MM481 epithelioid mesothelioma, MM487 and MM491 biphasic mesothelioma, MM432 and MM472 sarcomatoid mesothelioma, MM487Luc (luciferase-expressing) biphasic mesothelioma, U87MG-Luc glioma, U937 histiocytic lymphoma, RAJI, DG-75, and RAMOS Burkitt lymphoma, JECO-1, MAVER-2, MINO, REC-1, and Z-138 mantle cell lymphoma, KM-H2 and L-428 Hodgkin lymphoma, OCI-LY3 and U-2932 diffuse large B-cell lymphoma, NB4 promyelocytic leukemia (APL).

### Cell cultures

NCI-H460, H460-R9A, A2780, A2780-Dx, NB4, U937, RAJI, REC-1, DG-75, U-2932, KM-H2, L-428 cells were cultured in RPMI-1640 medium supplemented with 10 % FBS, 2 mM L-glutamine and gentamicin sulphate. MDA-MB436 cells were cultured in DMEM medium supplemented with 10 % FBS, 2 mM L-glutamine and gentamicin sulfate. MM288, MM317, MM404, MM473, MM481, MM487, MM491, MM432, MM472 cells were cultured in Ham's F-10 Nutrient Mixture supplemented with 10 % FBS, 2 mM L-glutamine, and gentamicin sulphate, MM487Luc cells were cultured in Ham's F-10 Nutrient Mixture supplemented with 10 % FBS, 2 mM L-glutamine, and G418 antibiotic. U87MG-Luc cells were cultured in EMEM medium supplemented with 10 % FBS, 2 mM L-glutamine and gentamicin sulfate. RAMOS, OCI-LY3, JECO-1, MAVER-2, MINO cells were cultured in RPMI-1640 medium supplemented with 20% FBS, 2 mM L-glutamine and gentamicin sulfate. Z-138 cells were cultured in IMDM medium supplemented with 10 % FBS, L-glutamine and gentamicin sulfate.

Cells were maintained in a 37°C incubator with saturated humidity and an atmosphere of 95% air and 5% CO2, and were sub-cultured every 2-3 days.

### Experimental Design

The anti-proliferative activity of a number of new atypical retinoids (AR) was assessed on various human cell lines from solid and hematological cancers.

To this end, cells in logarithmic phase of growth were seeded in 96-wells plastic plates, incubated overnight in culture medium, and treated with scalar concentrations of the compounds for either 24 h followed by 48 h recovery in drug-free-medium, or 72 h. Cell survival was finally assayed by the SRB (sulphate reducing bacterial) or MTT test and the IC50 value (drug concentration inhibiting 50% of cell growth) calculated by the ALLFIT program.

### Results

The following Tables 1-6 report examples of anti-proliferative activity of selected compounds.

### EXAMPLE 30

Tumor cells were exposed for 24 h to new atypical retinoids (AR) and the anti-proliferative activity assessed upon 48 h recovery in drug-free medium with the SRB assay. Results are reported in the following table 1.

**Table 1. Human NCI-H460 NSCLC exposed to new atypical retinoids**

| Compound | **IC₅₀ ± SD** (µM) |
|---|---|
| | |
| 1 **(AB461)** | ≤ 10 |
| 2 **(RC1120)** | ≤ 1 |
| 3 **(RC1151)** | < 10 |
| 4 **(RC1152)** | < 10 |
| 5 **(RC1193)** | ≤ 10 |
| 6 **(RC1190)** | ≤ 10 |
| 7 **(RC1191)** | ≤ 10 |
| 8 **(AB472)** | < 5 |
| 9 **(AB473)** | ≤ 10 |
| 10 **(AB474)** | ≤ 10 |
| 11 **(RC1189)** | ≤ 10 |
| 12 **(RC1171)** | ≤ 10 |
| 13 **(RC1174)** | < 5 |
| 14 **(AB486)** | ≤ 10 |
| 15 **(RC1237)** | < 10 |
| 16 **(RC1230)** | < 10 |
| 17 **(RC1243)** | < 5 |
| 18 **(RC1236)** | ≤ 10 |
| 19 **(RC1238)** | ≤ 10 |
| 20 **(RC1277)** | ≤ 10 |
| 27 **(RC1207)** | ≤ 10 |
| 29 **(MIR002)** | ≤ 0.5 |

### EXAMPLE 31

Tumor cells were chronically treated with various atypical retinoids and the anti-proliferative activity assessed upon 72 h with the SRB assay. Results are reported in the following table 2.

**Table 2. Human NCI-H460 NSCLC chronically exposed to new atypical retinoids**

| Compound | **IC₅₀ ± SD** (µM) |
|---|---|
| | |
| 21 **(RC1315)** | < 5 |
| 22 **(RC1375)** | < 5 |
| 23 **(RC1268)** | ≤ 5 |
| 24 **(RC1363)** | ≤ 10 |
| 25 **(RC1338)** | < 5 |
| 26 **(AB514)** | < 0.1 |
| 29 (MIR002) | < 0.5 |

### EXAMPLE 32

Tumor cells were exposed for 24 h to new atypical retinoids (AR) and the anti-proliferative activity assessed upon 48 h recovery in drug-free medium with the SRB assay. RI = Resistance Index (ratio between the IC50 values of resistant and sensitive cells). Results are reported in the following table 3.

**Table 3. Human Adarotene-resistant NCI-H460 (R9A) NSCLC, MM487-Luc mesothelioma, parent A2780 and A2780-Dx multidrug-resistant ovarian cancer cell lines, U87MG glioblastoma cells exposed to new atypical retinoids.**

| | **IC₅₀ ± SD** (µM) | | | | | |
|---|---|---|---|---|---|---|
| **Compound** | **H460-R9A** | **MM487-Luc** | **U87MG** | **A2780** | **A2780-Dx** | **RI** |
| **Adarotene (ST1926)** | 26,9± 0.7 | 9.2 ± 0.3 | 1.63 ± 0.1 | | | |
| **21 (RC1315)** | 8.3± 0.2 | 1.7 ± 0.08 | | 4.2± 0.3 | 3.9± 0.1 | 0.9 |
| **2 (RC1120)** | | | | 1.1 ± 0.5 | 6.0 ± 1.3 | 4.9 |
| **22 (RC1375)** | 5.9± 0.1 | 1.9± 0.2 | 5.5 ± 0.9* | 3.7± 0.3 | 4.5± 0.07 | 1.3 |
| **13 (RC1174)** | 7.5± 0.2 | 10.4 ± 0.3 | | 4.7 ± 0.5 | 12.1 ± 2.7 | 2.6 |
| **15 (RC1237)** | 13.5 ± 0.5 | | | 9.6 ± 0.2 | 5.5 ± 0.2 | 0.6 |
| **17 (RC1243)** | | | | 5.4 ± 0.7 | 2.5 ± 0.2 | 0.5 |
| **8 (AB472)** | 9.4 ± 0.3 | 1.2 ± 0.2 | | 1.2 ± 0.1 | 1.4 ± 0.08 | 1.2 |
| **29 (MIR002)** | 2.8 ± 0.3 | 4.5± 0.3 | 1.1 ± 0.3 | 0.6 ± 0.01 | 0.41 ± 0.005 | 0.7 |

As described by Zuco V. et al in Neoplasia, 2005 Jul;7(7):667-77, H460-R9A are about 70 fold resistant with respect to parental H460 to Adarotene. Surprisingly, the new retinoid derivatives showed a significantly increase of cytotoxic activity against this cell line compared to Adarotene. Ovarian cancer A2780 and its clone derivative A2780DX (Doxorubicin resistant) which overexpress P-glycoprotein (PGP), were used to evaluate the possibility of these new molecules to be absorbed at intestinal level. In fact, the overexpression of PGP, as well known, drastically reduces the possibility, after oral administration, of absorption of several chemotherapeutic agents (i.e. Paclitaxel, doxorubicin etc.). Since the IC50 of the tested compounds in A2780 and A2780DX, have similar values we conclude that they are not PGP substrate and they could be administrate to the patients via oral route. Moreover, the antitumor activity of RC1375 and MIR002 on Glioblastoma tumor cells (U87MG) suggest the use of this class of compounds in intracranial tumors including Glioma.

### EXAMPLE 33

Tumor cells were exposed for 24 h to new atypical retinoids (AR) and the anti-proliferative activity assessed upon 48 h recovery in drug-free medium with the MTT assay. Result are reported in the following table 4.

**Table 4. Human hematological cancer cell lines exposed to new atypical retinoids.**

| | **IC50 ± SD (µM)** | | |
|---|---|---|---|
| | **Adarotene** | **2 (RC1120)** | **8 (AB472)** |
| Cell lines | | | |
| **RAJI** Burkitt lymphoma | 1.0 ± 0.2 | 0.42 ± 0.1 | |
| **U937** Histiocytic lymphoma | | 1.8 ± 0.0 | |
| **DG-75** Burkitt lymphoma | | 0.55 ± 0.1 | 0.71 ± 0.2 |
| **RAMOS** Burkitt lymphoma | | 2.5 ± 0.7 | |
| **KM-H2** Hodgkin lymphoma | | 2.1 ± 0.6 | |
| **L-428** Hodgkin lymphoma | | 1.77 ± 0.5 | |
| **U-2932** ABC-DLBCL | | 1.26 ± 0.3 | |
| **OCI-LY3** ABC-DLBCL | | 1.5 ± 0.6 | 1.0 ± 0.2 |
| **JECO-1** mantle cell lymphoma | | | 1.0 ± 0.2 |
| **MINO** mantle cell lymphoma | | 1.1 ± 0.1 | |
| **REC-1** mantle cell lymphoma | | 1.2 ± 0.2 | |
| **Z-138** mantle cell lymphoma | | 0.47 ± 0.08 | 0.25 ± 0.02 |
| **NB4** (pro-myelocytic. leukemia) | | | 0.37 ± 0.08 |

### EXAMPLE 34

### Evaluation of antitumor activity of compounds 2 (RC1120) and 22 (RC1375) on xenograft mouse model

### MATERIALS AND METHODS

### Animals

CD-1 nude and NOG mice, females, 4-5 weeks-old, were housed under specific-pathogen-free conditions in individually ventilated cages. Ethics approval was obtained from the Italian Ministry of Health and all experiments were in accordance with the European guidelines for the care and use of laboratory animals.

### Cell cultures and tumor implants

Cells were cultured in appropriated media and expanded in a 37°C incubator with saturated humidity and an atmosphere of 95% air and 5% CO₂, and were sub-cultured every 2-3 days. Before the injection in mice, cells were checked with MycoAlert® Mycoplasma Detection Kit (Lonza) to exclude mycoplasma contamination, counted by trypan blue dye exclusion, evaluated for cell viability (> 95%).

*H-460 lung cancer cells* were resuspended at a concentration of 25x106/ml. 5x10⁶ tumor cells were suspended in 0.2 ml of M199, then inoculated subcutaneously on the right flank of each animal.

*Human Liver Hepatocarcinoma-Luc* cells *(HepG2-Luc)* were re-suspended at the concentration of 1x10⁶ cells/40µl M199 medium. U87MG-luc Human Brain Glioblastoma cells were resuspended in M199 medium at the concentration of 3x10⁵/5µL, then orthotopically injected in the right lobe of brain with infusion of 1µl/min (with Hamilton syringe).

### Orthotopic mesothelioma model

*(MM487-Luc)* were re-suspended at the concentration of 1x10⁶ cells/40µl M199 medium., then orthotopically injected intrapeulically with infusion of 1µl/min (with Hamilton syringe).

*Orthotopic glioma murine model.* 1x10⁶ Brain glioblastoma bioluminescent cells (U-87 MG) were injected intracranically and tumor growth was evaluated through IVIS imaging system.

### Reagents and in vivo treatments

Different concentrations of compounds 2 (RC1120) and 22 (RC1375) were diluted in 10% 1:1 absolute Ethanol-Cremophor solution in saline. Compounds and vehicle were administered IP or OS (gavage) at the volume of 5ml/kg.

### Measurements and in vivo imaging

Mice were weighed twice a week. Subcutaneous tumor masses were measured biweekly by Caliper and tumor volume was calculated using the following formula: TV (mm³) = [length (mm) x width (mm)²]/2. For murine orthotopic xenograft models, tumor growth was evaluated weekly by using the IVIS Spectrum (PerkinElmer) in vivo technology system, through intraperitoneal injection of D-Luciferin (100µL/10gr, PerkinElmer). For each animal was evaluated the specific average radiance signal.

### Statistical analysis

Statistical analysis was performed by using U-Test (GraphPad Prism 6). Outliers were removed by using the Rout test (Q = 10%, GraphPad Prism 6).

### RESULTS

To evaluate the anti tumor efficacy of new synthetic retinoids, human tumors were xenografted subcutaneously or orthotopically in nude mice.

**2 (RC1120)** effectiveness was evaluated on lung cancer model (NCI-H460) xenografted s.c. while hepatocellular carcinoma (Hep G2) was transplanted ortotopically in mice liver orthotopic model.

In particular for lung cancer model, H460 cells were injected into the right flank of CD-1 nude mice. 5 days after cells injection, tumor volume was measured by caliper and mice were allocated in experimental groups on the basis of tumor volumes. Particularly, mice were allocated in three different groups: animals of control group 1 were treated with vehicle while in the group 2 the animals were treated with compound **2 (RC1120),** at 30mg/kg, by gavage (qdx5d). Tumor volume was measured during the treatment period to evaluate the inhibitory effect of 2 (RC 1120) on the tumor growth. At the end of the experiment a tumor volume inhibition (TVI%) of about 46 % was observed in mice treated with 2 (RC1120) compared to animals treated with vehicle only.

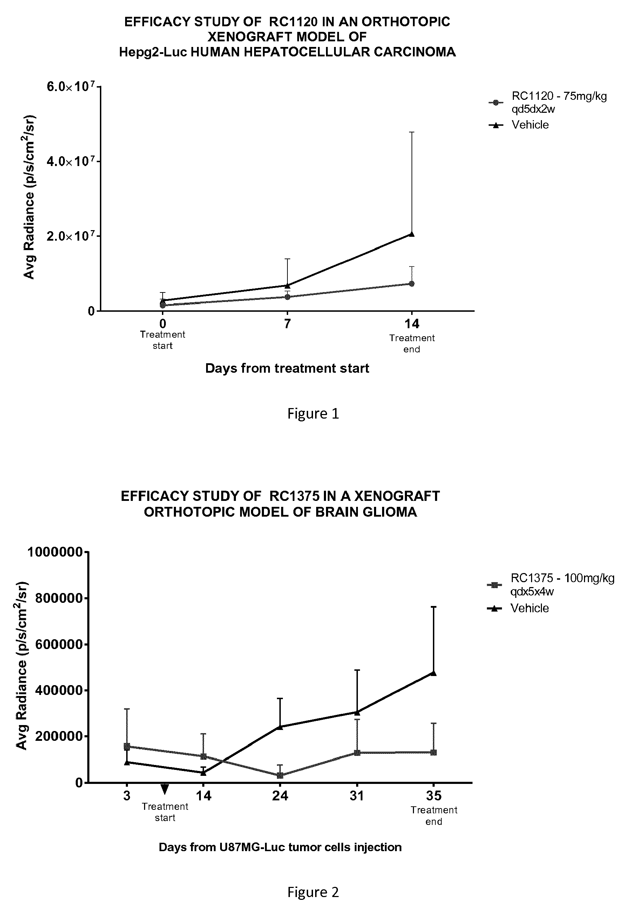

An improved effect was observed on an hepatocellular carcinoma orthotopic murine model. In this case, luciferase expressing HepG2 cells were injected directly in liver of NOG mice and tumor growth was evaluated as bioluminescence increase using IVIS imaging system. After few days from injection, to allow engraftment of cells, mice were sorted for BLI signal and orally treated with **2 (RC1120)** (75mg/kg) or vehicle only as negative control, 5 days/week for 2 consecutive weeks. The tumor volume was measured as BLI signal (average radiance) during the experiment. As shown in figure 1, at the end of treatment compound 2 (RC1120) showed a significant antitumor efficacy, with a tumor volume inhibition of 72% compared with animals treated with vehicle only.

The antitumor activity of **8 (AB472)** was evaluated in orthotopic model of human mesothelioma named MM487. These cells were stable transfected with vector expressing luciferase. **8 (AB472)** was administered orally, every day, after two weeks from tumor injection, for 4 weeks at dose of 75mg/kg. Cisplatin (IP) was administered, two weeks after tumor injection, every week for 4 weeks at dose of 5 mg/Kg. As shown AB472, strongly reduces tumor growth and compared to Cisplatin (Cis) activity, the golden standard therapy for this tumor, possess a much higher antitumor activity.

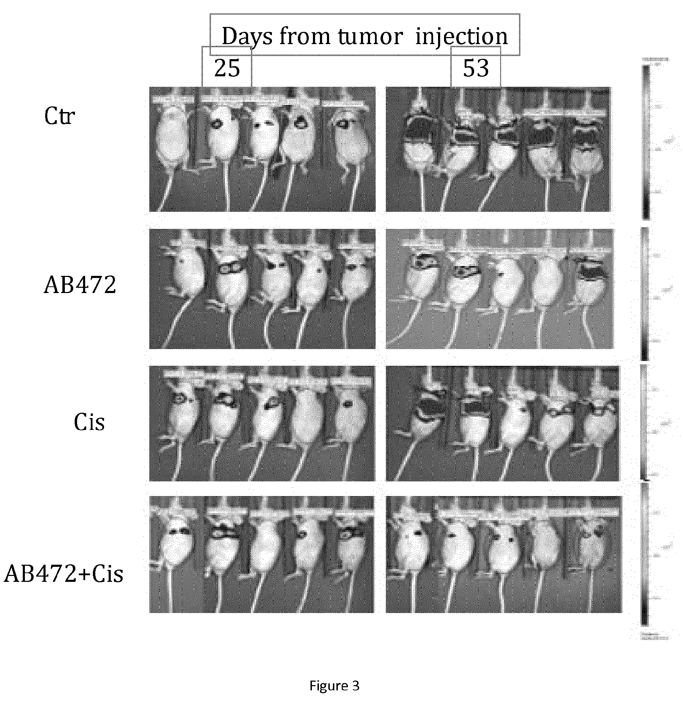

The combination of AB472 with Cis, resulted in a unexpected impressive synergic antitumor activity, showing a 95% of TVI compared to vehicle. The observation performed with the Ivis system detecting the luciferase activity of this tumor, reveals that AB472, as single agent, by inhibiting tumor growth in all mice treated, eradicate the tumor in one mice (Figure 3). Impressively, the combination of AB472+Cis completely block and reduces the mesothelioma growth comparing the day in which the treatments start (day25) and the end of experiment (day53).

The activity of compound **22 (RC1375)** was evaluated on glioma xenograft murine models. Brain glioblastoma bioluminescent cells (U-87 MG) were injected intracranically and tumor growth was evaluated through IVIS imaging system. After a week of injection, 22 (RC1375) (100mg/kg) was administered intraperitoneally to mice (qdx5x4w). As shown in Figure 2, treatment with compound 22 (RC1375) determines a significant tumor reduction with a TVI of 72%.

All the results are summarized in the table 5 below.

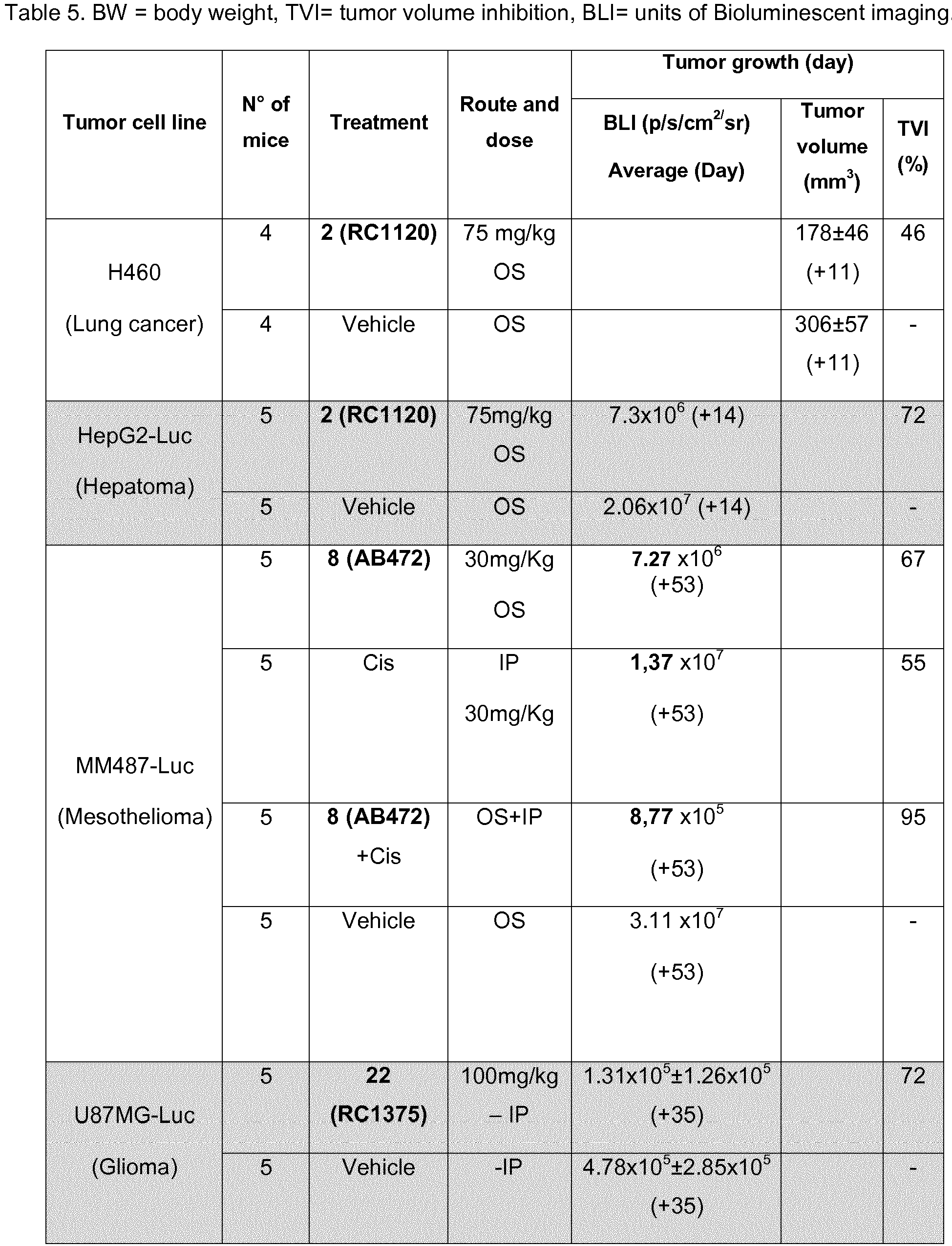

Taken together, the results from *in vitro* and *in vivo* experiments indicate that this new class of synthetic retinoids, including compounds 2 (RC1120), 22 (RC1375), 8 (AB472), 29 (MIR002), possesses a very large spectrum of anti-tumoral activity against several tumor histotypes suggesting their therapeutic use for the treatment of different human cancers. Moreover, the administration of these compounds did not show any negative effect on mice body weight, thus suggesting an high tolerability.

## Claims

1. A compound having the following formula (I): 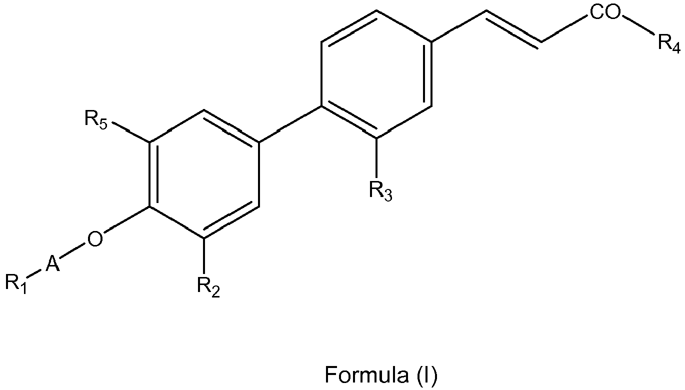 wherein:
A is selected from the group consisting of: alkylene, alkenylene and alkynylene, or is absent,
R₁ is selected from the group consisting of: H, OH, COOH, COO-alkyl, CONHOH, CONH₂, COCF₃, SH, SCOCH₃, CONHalkyl-S-S-alkyl, CONHalkyl-S-S-alkyl-NH₂, CONHalkyl-S-S-alkyl-NHCO-C(=NOH)alkylaryl wherein said aryl is optionally substituted with OH or halogen, COalkylCONHalkyl-S-S-alkyl-NHCO-C(=NOH)alkylaryl wherein said aryl is optionally substituted with OH or halogen, O-4-retinoic acid and O-4-N-(4-hydroxyphenyl)retinamide,
R₂ is selected from the group consisting of: H, alkyl, OH, O-alkyl, O-alkenyl, halogen, CN, CHO, COalkyl, COOH, COOalkyl and CONH₂,
R₃ is selected from the group consisting of: H, alkyl, alkenyl, oxo-alkenyl, hydroxyiminoalkenyl, OH, alkoxy, cycloalkyloxy, arylalcoxy, heterocycloalcoxy, carboxyalcoxy, CH₂R₆, COR₇, CN, CH=NOH, CH=NOalkylCOOH, CH=NOaryl and CH=NOalkylaryl,
wherein R₆ is selected from the group consisting of: OH, alkoxy, NH₂, NH-alkyl, N-dialkyl, NHCOOalkyl, NHCOalkyl, aryl, heterocycloalkyl, guanidino optionally substituted with an alkyl or a COOalkyl group, biguanido optionally substituted with an alkyl or an arylalkyl group,
and wherein R₇ is selected from the group consisting of: H, OH, O-alkyl, NH₂, NHOH, NHaryl, NHalkyl optionally terminally substitued with NH₂ or CONHOH,
R₄ is selected from the group consisting of: OH, O-alkyl, NH₂, NHaryl, NHalkyl, Ndialkyl, NHOH, NHNH₂ and CF₃, and
R₅ is selected from the group consisting of: alkyl, cycloalkyl, azacycloalkyl, oxacycloalkyl, aryl, heteroaryl, alkylamminomethyl, arylakylaminomethyl, heteroarylalkylaminomethyl, wherein each of said groups can be optionally substituted with OH, Oalkyl, halogen, NO₂ or NH₂, and each of cycloalkyl, azacycloalkyl, oxacycloalkyl, aryl, heteroaryl group can be substituted with alkyl,
any nitrogen atom optionally bearing a protective group,
wherein alkyl is a C₁-C₂₀ linear or branched alkyl group,
alkenyl is a C₂-C₂₀ linear or branched alkyl group,
cycloalkyl is a saturated or partially unsaturated C₃-C₁₀ carbocyclic group comprising one or more condensed rings,
aryl is an aromatic group comprising one or more condensed aromatic rings, heterocycloalkyl and heteroaryl are said cycloalkyl or aryl rings containing one or more heteroatoms selected from the group consisting of N, O and S,
amino is a group NR'R" wherein each of R' and R" can independently be H or alkyl, provided that when R₃ is H, R₁ is not H,
the enantiomers, diastereoisomers, and mixtures thereof,
or a pharmaceutically acceptable salt, hydrate or solvate thereof,
provided that it is not the compound (E)-3-(3'-Adamantan-1-yl-4'-carbamoylmethoxybiphenyl-4-yl)-acrylic acid.

2. The compound according to claim 1, wherein R₅ is a cycloalkyl.

3. The compound according to claim 1 or 2, wherein R₅ is an adamantyl group.

4. The compound according to anyone of claims 1-3, wherein R₄ is OH or O-alkyl.

5. The compound according to anyone of claims 1-4, wherein A is an alkyl and R₁ is H.

6. The compound according to anyone of claims 1-5, wherein A is absent and R₁ is H.

7. The compound according to anyone of claims 1-6, which is selected from the group consisting of:
3-[3'-Adamantan-1-yl-2-(tert-butoxycarbonylamino-methyl)-4'-hydroxy-biphenyl-4-yl]-acrylic acid (1)(AB461)
3-(3'-adamantan-1-yl-2-aminomethyl-4'-hydroxy-biphenyl-4-yl)-acrylic acid trifluoroacetate (2) (RC 1120)
3-(3'-adamantan-1-yl-4'-hydroxy-2-morpholin-4-ylmethyl-biphenyl-4-yl)-acrylic acid hydrochloride (3) (RC 1151)
3-(3'-adamantan-1-yl-4'-hydroxy-3-(4-methyl-pi perazin-1-ylmethyl)-biphenyl-4-yl)-acrylic acid hydrochloride (4) (RC 1152)
3-[3'-adamantan-1-yl-2-(4-amino-butylcarbamoyl)-4'-hydroxy-biphenyl-4-yl]-acrylic acid trifluoroacetate (5) (RC 1193)
3-(3'-adamantan-1-yl-2-ditert-butoxycarbonylguanidinomethyl-4'-hydroxy-biphenyl-4-yl)-acrylic acid trifluoroacetate (6) (RC 1190)
3-(3'-adamantan-1-yl-2-guanidinomethyl-4'-hydroxy-biphenyl-4-yl)-acrylic acid trifluoroacetate (7) (RC 1191)
3-[3'-adamantan-1-yl-4'-hydroxy-2-(hydroxyimino-methyl)-biphenyl-4-yl]-acrylic acid (8) (AB 472)
3-[3'-adamantan-1-yl-4'-hydroxy-2-(tert-butoxyimino-methyl)-biphenyl-4-yl]-acrylic acid (9) (AB 473)
3-[3'-adamant-1-yl-4'-hydroxy-2-methoxyimino-methyl-biphenyl-4-yl]-acrylic acid. (10) (AB 474)
3-[3'-Adamantan-1-yl-2-(4-tert-butoxycarbonylamino-butylcarbamoyl)-4'-hydroxybiphenyl-4-yl]-acrylic acid (11) (RC1189)
3-[3'-adamantan-1-yl-2-(tert-butoxycarbonylaminomethyl)-4'-methoxy-biphenyl-4-yl]-acrylic acid (12) (RC 1171)
3'-Adamantan-1-yl-4-(2-carboxy-vinyl)-4'-methoxy-biphenyl-2-yl-methyl-ammonium trifluoroacetate (13) (RC 1174)
3-[3'-Adamant-1-yl-4'-hydroxy-2-carboxymethoxyimino-methyl-biphenyl-4-yl]-acrylic acid. (14) (AB 486)
3-[3'-Adamantan-1-yl-4'-methoxy-2-(formyl)-biphenyl-4-yl]-acrylic acid (15) (RC 1237)
3-[3'-Adamantan-1-yl-4'-methoxy-2-(hydroxyimino-methyl)-biphenyl-4-yl]-acrylic acid (16) (RC1230)
3-[3'-Adamantan-1-yl-4'-methoxy-2-(hydroxymethyl)-biphenyl-4-yl]-acrylic acid (17) (RC 1243)
3-[3'-Adamantan-1-yl-4'-methoxy-2-(3-oxo-but-1-enyl)-biphenyl-4-yl]-acrylic acid (18) (RC1236)
3-[3'-Adamantan-1-yl-2-(3-hydroxyimino-but-1-enyl)-4'-methoxy-biphenyl-4-yl]-acrylic acid (19) (RC1238)
3-(3'-Adamantan-1-yl-2-carbamoyl-4'-methoxy-biphenyl-4-yl)-acrylic acid (20) (RC 1277)
3-[3'-Adamantan-1-yl-4'-(3-hydroxycarbamoyl-propoxy)-biphenyl-4-yl]-acrylic acid (21) (RC 1315)
3-[3'-Adamantan-1-yl-4'-(4-hydroxycarbamoyl-butoxy)-biphenyl-4-yl]-acrylic acid (22) (RC 1375)
3-[3'-Adamantan-1-yl-4'-(7-hydroxycarbamoyl-heptyloxy)-biphenyl-4-yl]-acrylic acid (23) (RC 1268)
2-(2-{4-[3-Adamantan-1-yl-4'-(2-carboxy-vinyl)-biphenyl-4-yloxy]-butyrylamino}-ethyldisulfanyl)-ethyl-ammonium; trifluoroacetate (24) (RC 1363)
3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propoxy]-biphenyl-4-yl}-acrylic acid (25) (RC 1338)
3-{3'-Adamantan-1-yl-4'-[3-(2-{2-[3-(3-bromo-4-hydroxy-phenyl)-2-hydroxyimino-propionylamino]-ethyldisulfanyl}-ethylcarbamoyl)-propionyloxy]-biphenyl-4-yl}-acrylic acid (26) (AB 514)
3-[3'-Adamantan-1-yl-4'-hydroxy-2-(7-hydroxycarbamoyl-heptylcarbamoyl)-biphenyl-4-yl]-acrylic acid (27) (RC 1207)
3-[3'-Adamantan-1-yl-4'-(4-carboxy-butoxy)-biphenyl-4-yl]-acrylic acid (28)
3-(3'-Adamantan-1-yl-4'-hydroxycarbamoylmethoxybiphenyl-4-yl)-acrylic acid (29)
3-[3'-(1,5-Diaza-bicyclo[3.3.1]non-9-yl)-4'-(4-hydroxycarbamoyl-butoxy)-biphenyl-4-yl]-acrylic acid (30)
3-[4'-(4-Hydroxycarbamoyl-butoxy)-3'-(1,3,5,7-tetraaza-tricyclo[3.3.1.13,7]dec-2-yl)-biphenyl-4-yl]-acrylic acid (31)
3-[4'-(4-Hydroxycarbamoyl-butoxy)-3'-(1-methyl-cyclohexyl)-biphenyl-4-yl]-acrylic acid (32)
3-Phenyl-4'-(4-hydroxycarbamoyl-butoxy)-biphenyl-4-yl]-acrylic acid (33)
3-(4'-(4-Hydroxycarbamoyl-butoxy)-3'-{[(naphthalen-2-ylmethyl)-amino]-methyl}-biphenyl-4-yl)-acrylic acid (34)
3-(4'-(4-Hydroxycarbamoyl-butoxy)-3'-{[(quinolin-7-ylmethyl)-amino]-methyl}-biphenyl-4-yl)-acrylic acid (35)
3-(4'-(4-Hydroxycarbamoyl-butoxy)-3'-{[(8-hydroxy-5-nitro-quinolin-7-ylmethyl)-amino]-methyl}-biphenyl-4-yl)-acrylic acid (36), and
9-(3-{4-[3-Adamantan-1-yl-4'-(2-carboxy-vinyl)-biphenyl-4-yloxy]-butoxy}-2,6,6-trimethyl-cyclohex-1-enyl)-3,7-dimethyl-nona-2,4,6,8-tetraenoic acid (37).

8. The compound of anyone of claims 1-7 for use as a medicament.

9. The compound of anyone of claims 1-7 for use in the treatment of a disease related to altered angiogenesis.

10. The compound of anyone of claims 1-7 for use in the treatment of a disease selected from the group consisting of arthritic pathology, tumour, metastatization, diabetic retinopathy, psoriasis and chronic inflammatory disease.

11. The compound for the use according to claim 10, wherein said tumour is selected from the group consisting of sarcoma, carcinoma, carcinoid, mesothelioma, lymphoma, bone tumour, neuroendocrine tumour, lymphoid leukaemia, myeloid leukaemia, monocytic leukaemia, megakaryocytic leukaemia, acute promyelocytic leukaemia, Hodgkin's disease, lung tumour, hepatoma, mesothelioma, intracranial tumor and glioma.

12. A pharmaceutical composition comprising as active ingredient a compound of anyone of claims 1-7 and at least one pharmaceutically acceptable vehicle and/or excipient.

13. The pharmaceutical composition according to claim 12 further comprising one or more chemotherapeutic agents.

14. The pharmaceutical composition according to claim 13, wherein said further chemotherapeutic agent is a platinum-based chemotherapeutic agent.
